# EUROPEAN PATENT APPLICATION

(11) **EP 4 530 821 A1**
(43) Date of publication of application: **02.04.2025**
(21) Application number: 23859445.1
(22) Date of filing: 31.08.2023
(51) Int. Cl.: G06F 3/16, G06N 3/08

(54) **VOLUME ADJUSTMENT METHOD, ELECTRONIC DEVICE AND SYSTEM**

(30) Priority: 01.09.2022 CN 202211067066
(71) Applicant: Huawei Technologies Co., Ltd., Shenzhen, Guangdong 518129 (CN)
(72) Inventor: BAO, Guangzhao, Shenzhen, Guangdong 518129 (CN); WANG, Chunpeng, Shenzhen, Guangdong 518129 (CN); PANG, Lichen, Shenzhen, Guangdong 518129 (CN)
(74) Representative: Epping - Hermann - Fischer
(86) International application number: PCT/CN2023/116067
(87) International publication number: WO 2024/046416

(57) **Abstract**

This application provides a volume adjustment method, an electronic device, and a system. The method includes: In response to a user operation of starting a hearing test, an electronic device separately plays preset audio at a plurality of volumes, where the plurality of volumes include a first volume; determines, based on a confirmation operation for the first volume, that a hearing level of a user is a first hearing level corresponding to the first volume; when playing a downlink signal by using a sound-emitting apparatus, collects the downlink signal and an ambient noise signal by using at least one microphone; and adjusts a play volume of the sound-emitting apparatus from a first sound level to a second sound level based on the first hearing level when at least one of the collected downlink signal and the collected ambient noise signal changes. Embodiments of this application are implemented, so that the electronic device can adjust the play volume of the sound-emitting apparatus based on the ambient noise signal, the downlink signal, and the hearing level of the user, to improve user experience.

## Description

This application claims priority to Chinese Patent Application No. 202211067066.3, filed with the China National Intellectual Property Administration on September 1, 2022 and entitled "VOLUME ADJUSTMENT METHOD, ELECTRONIC DEVICE, AND SYSTEM", which is incorporated herein by reference in its entirety.

### TECHNICAL FIELD

Embodiments of this application relate to terminal technologies, and in particular, to a volume adjustment method, an electronic device, and a system.

### BACKGROUND

In daily life, when a user uses a terminal device (including but not limited to a mobile phone, glasses, open-back headphones, or the like) to make a call or listen to music in a public scenario (such as an elevator or a conference room), content of the call or music is often heard by a person nearby. This causes a risk of personal privacy leakage or interference to others.

How to intelligently adjust a play volume of the terminal device is a current and future research direction.

### SUMMARY

This application provides a volume adjustment method, an electronic device, and a system. In the volume adjustment method, a play volume of a downlink signal can be adjusted based on an ambient noise signal, the downlink signal, and a hearing level of a user, to improve user experience.

According to a first aspect, an embodiment of this application provides a volume adjustment method. The method is applied to an electronic device. The electronic device includes a sound-emitting apparatus and at least one microphone. The method includes:

In response to a user operation of starting a hearing test, the electronic device separately plays preset audio at a plurality of volumes, where the plurality of volumes include a first volume.

The electronic device determines, based on a confirmation operation for the first volume, that a hearing level of a user is a first hearing level corresponding to the first volume.

When playing a downlink signal by using the sound-emitting apparatus, the electronic device collects the downlink signal and an ambient noise signal by using the at least one microphone.

The electronic device adjusts a play volume of the sound-emitting apparatus from a first sound level to a second sound level based on the first hearing level when at least one of the collected downlink signal and the collected ambient noise signal changes.

In this embodiment of this application, the electronic device may determine the hearing level of the user based on the confirmation operation of the user in the hearing test, and further adjust, based on the hearing level of the user, the play volume of the sound-emitting apparatus when at least one of the collected downlink signal or the collected ambient noise signal changes. It can be learned that the play volume is a volume determined based on comprehensive consideration of the ambient noise signal, the downlink signal, and the hearing level of the user, so that an individual differential requirement of the user can be met, impact of ambient noise can be reduced, and user experience can be improved.

With reference to the first aspect, in a possible implementation, a higher first volume indicates a higher level of the second sound level.

In this embodiment of this application, a higher volume of the first volume selected by the user in the hearing level test indicates a higher sound level, at which the electronic device plays the downlink signal, in a process in which the user actually uses the electronic device. According to the method, hearing requirements of different users can be met.

With reference to the first aspect, in a possible implementation, the first volume is a comfortable volume for the user or a lowest volume at which the user is able to clearly hear in a current environment.

In this embodiment of this application, the user may select the first volume based on a personal requirement of the user in the hearing level test. For example, if the user expects that a volume of the downlink signal played by the electronic device is the comfortable volume for the user, the user may determine the comfortable volume for the user in the current environment as the first volume. For another example, if the user expects that the volume of the downlink signal played by the electronic device is the lowest volume at which the user is able to clearly hear, the user may determine the lowest volume at which the user is able to clearly hear in the current environment as the first volume. According to the method, different volume requirements of the user can be met.

With reference to the first aspect, in a possible implementation, greater energy of a changed ambient noise signal indicates a higher level of the second sound level.

In this embodiment of this application, the electronic device may automatically adjust a sound level of the sound-emitting apparatus when energy of the ambient noise signal changes. For example, if the energy of the ambient noise signal increases, that is, the noise is enhanced, the electronic device may adjust the sound level of the sound-emitting apparatus to a high level, to avoid a case in which the user cannot clearly hear the downlink signal due to the ambient noise signal.

With reference to the first aspect, in a possible implementation, that the electronic device adjusts a play volume of the sound-emitting apparatus from a first sound level to a second sound level when at least one of the downlink signal and the ambient noise signal changes includes:

The electronic device adjusts the play volume of the sound-emitting apparatus to the second sound level when a difference between the first sound level and the second sound level equals a first preset quantity of levels.

With reference to the first aspect, in a possible implementation, that the electronic device adjusts a play volume of the sound-emitting apparatus from a first sound level to a second sound level when at least one of the downlink signal and the ambient noise signal changes includes:

The electronic device adjusts the play volume of the sound-emitting apparatus to a third sound level when the difference between the first sound level and the second sound level equals a second preset quantity of levels. The third sound level is higher than the first sound level and lower than the second sound level.

In this embodiment of this application, the electronic device may gradually adjust the first sound level to the second sound level when the difference between the first sound level and the second sound level equals the second preset quantity of levels. Assuming that the first sound level is lower than the second sound level, the electronic device may first adjust the play volume of the sound-emitting apparatus to the third sound level, where the third sound level is higher than the first sound level and lower than the second sound level; and then adjust the play volume of the sound-emitting apparatus from the third sound level to the second sound level. Herein, a case in which the third sound level is used as an intermediate adjustment is merely used as an example for description. In another embodiment, alternatively, the sound-emitting apparatus may be adjusted from the first sound level to the second sound level through a plurality of intermediate adjustments. An adjustment in a case in which the first sound level is higher than the second sound level is similar to the foregoing adjustment. The first sound level may be gradually lowered to the second sound level. According to the method, uncomfortable hearing experience of the user caused by a sudden volume adjustment can be avoided.

With reference to the first aspect, in a possible implementation, the method further includes:

In response to a user operation for a first application, the electronic device plays, in a first environment condition at a second volume by using the sound-emitting apparatus, a downlink signal corresponding to the first application.

In response to a user operation for a second application, the electronic device plays, in the first environment condition at a third volume by using the sound-emitting apparatus, a downlink signal corresponding to the second application.

The second volume is not equal to the third volume.

In this embodiment of this application, the electronic device may have different volume adjustments for different applications. According to the method, hearing requirements of the user for the different applications can be met.

For example, the different applications correspond to different target models, and sound levels output by the different target models in a same environment condition are different. In this case, volumes of downlink signals played by the electronic device are different.

With reference to the first aspect, in a possible implementation, the first application is a call-type application, the second application is a music-type application, and the second volume is greater than the third volume.

In this embodiment of this application, considering that the user has a high requirement for clear hearing of the call-type application, a volume of a downlink signal of the call-type application is adjusted to be great. Therefore, in a same environment, a play volume of the electronic device for the call-type application is greater than a play volume of the music-type application.

With reference to the first aspect, in a possible implementation, the method further includes:

The electronic device determines a preset model corresponding to the first hearing level as a target model, where the target model is obtained through training based on user data of a user that meets the first hearing level, and the user data includes a target sound level of the user at the first hearing level in a second environment condition and an energy difference between a downlink signal and an ambient noise signal that are collected in the second environment condition.

The electronic device inputs an energy difference between a changed downlink signal and the changed ambient noise signal into the target model, to obtain the second sound level.

With reference to the first aspect, in a possible implementation, the changed downlink signal includes N first subband signals, the changed ambient noise signal includes N second subband signals, and N is a positive integer. The N first subband signals are in a one-to-one correspondence with the N second subband signals. A first subband signal and a second subband signal that correspond to each other form a group of signals.

The energy difference between the changed downlink signal and the changed ambient noise signal includes energy differences of N groups of signals.

In this embodiment of this application, the energy difference between the changed downlink signal and the changed ambient noise signal is used as input data of the target model. The energy difference may include energy differences between frequency band-specific signals of the noise signal and frequency band-specific signals of the downlink signal. If a decibel value of a signal in any frequency band in the frequency band-specific signals of the downlink signal is greater than a masking threshold in masking effect, the downlink signal may be heard by the user. The method is based on the masking effect, so that it can be ensured that an adjusted volume meets a hearing requirement of a human ear in an adjustment process, to avoid interference of a masking sound.

With reference to the first aspect, in a possible implementation, the user data further includes a play volume of the sound-emitting apparatus at previous several moments of the target sound level. That the electronic device inputs an energy difference between a changed downlink signal and the changed ambient noise signal into the target model, to obtain the second sound level includes:

The electronic device inputs the energy difference between the changed downlink signal and the changed ambient noise signal and the play volume of the sound-emitting apparatus at the previous several moments into the target model, to obtain the second sound level.

With reference to the first aspect, in a possible implementation, after that the play volume of the sound-emitting apparatus is adjusted to the second sound level, the method further includes:

In response to an operation of adjusting a volume button by the user, the electronic device adjusts the play volume of the sound-emitting apparatus to the target sound level.

The electronic device stores the target sound level and the energy difference between the changed downlink signal and the changed ambient noise signal.

The electronic device trains the target model by using the target sound level and the energy difference between the changed downlink signal and the changed ambient noise signal as sample data.

In this embodiment of this application, the electronic device may collect the target sound level obtained through the adjustment during the operation of manually adjusting the volume button by the user and the volume difference, and train the target model by using the target sound level and the volume difference as the sample data. According to the method, the electronic device can increasingly conform to a user habit, to improve user experience.

With reference to the first aspect, in a possible implementation, the at least one microphone includes a first microphone and a second microphone. A distance between the first microphone and the sound-emitting apparatus is less than a distance between the second microphone and the sound-emitting apparatus. The first microphone is configured to collect the downlink signal, and the second microphone is configured to collect the ambient noise signal.

According to a second aspect, an embodiment of this application provides another volume adjustment method. The method is applied to an electronic device. The electronic device includes a sound-emitting apparatus and at least one microphone. The method includes:

When playing a downlink signal by using the sound-emitting apparatus, the electronic device collects the downlink signal and an ambient noise signal by using the at least one microphone.

The electronic device adjusts a play volume of the sound-emitting apparatus from a first sound level to a second sound level based on a first hearing level when at least one of the collected downlink signal and the collected ambient noise signal changes. The first hearing level is a hearing level corresponding to a user of the electronic device.

According to a third aspect, an embodiment of this application provides a volume adjustment apparatus. The apparatus includes a processing module, a sound-emitting apparatus, and at least one microphone.

The sound-emitting apparatus is configured to separately play preset audio at a plurality of volumes, where the plurality of volumes include a first volume.

The processing module is configured to determine, based on a confirmation operation for the first volume, that a hearing level of a user is a first hearing level corresponding to the first volume.

The at least one microphone is configured to: when the sound-emitting apparatus plays a downlink signal, collect the downlink signal and an ambient noise signal.

The sound-emitting apparatus is configured to adjust a play volume from a first sound level to a second sound level based on the first hearing level when at least one of the collected downlink signal and the collected ambient noise signal changes.

With reference to the third aspect, in a possible implementation, a higher first volume indicates a higher level of the second sound level.

With reference to the third aspect, in a possible implementation, the first volume is a lowest volume at which the user is able to clearly hear or a comfortable volume for the user in a current environment.

With reference to the third aspect, in a possible implementation, greater energy of a changed ambient noise signal indicates a higher level of the second sound level.

With reference to the third aspect, in a possible implementation, the sound-emitting apparatus is specifically configured to play the downlink signal at the second sound level when a difference between the first sound level and the second sound level equals a first preset quantity of levels.

With reference to the third aspect, in a possible implementation, the sound-emitting apparatus is specifically configured to: play the downlink signal at a third sound level when the difference between the first sound level and the second sound level equals a second preset quantity of levels. The third sound level is higher than the first sound level and lower than the second sound level.

With reference to the third aspect, in a possible implementation, the sound-emitting apparatus is configured to play, in a first environment condition at a second volume, a downlink signal corresponding to a first application.

The sound-emitting apparatus is configured to play, in the first environment condition at a third volume, a downlink signal corresponding to a second application, where the second volume is not equal to the third volume.

With reference to the third aspect, in a possible implementation, the first application is a call-type application, the second application is a music-type application, and the second volume is greater than the third volume.

With reference to the third aspect, in a possible implementation, the processing module is configured to:
determine a preset model corresponding to the first hearing level as a target model, where the target model is obtained through training based on user data of a user that meets the first hearing level, and the user data includes a target sound level of the user at the first hearing level in a second environment condition and an energy difference between a downlink signal and an ambient noise signal that are collected in the second environment condition; and
input an energy difference between a changed downlink signal and the changed ambient noise signal into the target model, to obtain the second sound level.

With reference to the third aspect, in a possible implementation, the changed downlink signal includes N first subband signals, the changed ambient noise signal includes N second subband signals, and N is a positive integer. The N first subband signals are in a one-to-one correspondence with the N second subband signals. A first subband signal and a second subband signal that correspond to each other form a group of signals.

The energy difference between the changed downlink signal and the changed ambient noise signal includes energy differences of N groups of signals.

With reference to the third aspect, in a possible implementation, the user data further includes a play volume of the sound-emitting apparatus at previous several moments of the target sound level.

The processing module is configured to input the energy difference between the changed downlink signal and the changed ambient noise signal and the play volume of the sound-emitting apparatus at the previous several moments into the target model, to obtain the second sound level.

With reference to the third aspect, in a possible implementation, the sound-emitting apparatus is configured to: in response to an operation of adjusting a volume button by the user, adjust the play volume of the sound-emitting apparatus to the target sound level.

The processing module is configured to store the target sound level and the energy difference between the changed downlink signal and the changed ambient noise signal.

The processing module is configured to train the target model by using the target sound level and the energy difference between the changed downlink signal and the changed ambient noise signal as sample data.

With reference to the third aspect, in a possible implementation, the at least one microphone includes a first microphone and a second microphone. A distance between the first microphone and the sound-emitting apparatus is less than a distance between the second microphone and the sound-emitting apparatus. The first microphone is configured to collect the downlink signal, and the second microphone is configured to collect the ambient noise signal.

According to a fourth aspect, an embodiment of this application provides an electronic device, including one or more functional modules. The one or more functional modules may be configured to perform the volume adjustment method in any one of the foregoing aspects or any possible implementation of any one of the foregoing aspects.

According to a fifth aspect, this application provides a computer storage medium, including computer instructions. When the computer instructions are run on an electronic device, a communication apparatus is enabled to perform the volume adjustment method in any one of the foregoing aspects or any possible implementation of any one of the foregoing aspects.

According to a sixth aspect, this application provides a computer program product. When the computer program product is run on a computer, the computer is enabled to perform the volume adjustment method in any one of the foregoing aspects or any possible implementation of any one of the foregoing aspects.

According to a seventh aspect, this application provides a chip, including a processor and an interface. The processor and the interface cooperate with each other, so that the chip performs the volume adjustment method in any one of the foregoing aspects or any possible implementation of any one of the foregoing aspects.

It may be understood that the electronic device provided in the fourth aspect, the computer-readable storage medium provided in the fifth aspect, the computer program product provided in the sixth aspect, and the chip provided in the seventh aspect are all configured to perform the methods provided in embodiments of this application. Therefore, for beneficial effect that can be achieved by the electronic device, the computer-readable storage medium, the computer program product, and the chip, refer to the beneficial effect in the corresponding methods. Details are not described herein again.

### BRIEF DESCRIPTION OF DRAWINGS

FIG. 1 is a diagram of windowing a voice signal according to an embodiment of this application;
FIG. 2 is a diagram of masking effect according to an embodiment of this application;
FIG. 3 is a diagram of a hardware structure of an electronic device 100 according to an embodiment of this application;
FIG. 4 is a diagram of a hardware form of a mobile phone according to an embodiment of this application;
FIG. 5 is a diagram of a hardware form of glasses according to an embodiment of this application;
FIG. 6 is a block diagram of a software structure of an electronic device 100 according to an embodiment of this application;
FIG. 7 is an overall schematic flowchart of a volume adjustment method according to an embodiment of this application;
FIG. 8 is a diagram of a correspondence between a preset model and a hearing level according to an embodiment of this application;
FIG. 9 is a diagram of a training process of a preset model according to an embodiment of this application;
FIG. 10 is a diagram of a training process according to an embodiment of this application;
FIG. 11 is an overall schematic flowchart of a volume adjustment method according to this application;
FIG. 12A to FIG. 12F are diagrams of some example interfaces in which a user performs a hearing test on an electronic device according to an embodiment of this application;
FIG. 13 is a diagram of performing frame segmentation and windowing on a signal collected by a bottom microphone according to an embodiment of this application;
FIG. 14 is a diagram of performing frame segmentation and windowing on a signal collected by a top microphone according to an embodiment of this application;
FIG. 15 is a diagram of subband segmentation processing according to an embodiment of this application;
FIG. 16 is a diagram of a correspondence between a sound level and a play volume according to an embodiment of this application;
FIG. 17 is a diagram in which an output sound level changes with an environment according to an embodiment of this application;
FIG. 18 is a diagram of comparison between play volumes of calls made by different electronic devices in a same environment according to an embodiment of this application;
FIG. 19 is a diagram of a volume adjustment process in a call process of a user according to an embodiment of this application;
FIG. 20 is a diagram of comparison between sound levels obtained through adjustments when an electronic device uses different applications according to an embodiment of this application;
FIG. 21 is a part of application interfaces of a call made by an electronic device in a first environment condition before self-learning according to an embodiment of this application; and
FIG. 22 is a part of application interfaces of a call made by an electronic device in a first environment condition after self-learning according to an embodiment of this application.

### DESCRIPTION OF EMBODIMENTS

Technical solutions according to embodiments of this application are clearly and completely described in the following with reference to accompanying drawings. In the descriptions of embodiments of this application, unless otherwise stated, "/" represents "or". For example, A/B may represent A or B. In this specification, "and/or" merely describes an association relationship between associated objects and represents that three relationships may exist. For example, A and/or B may represent the following three cases: Only A exists, both A and B exist, and only B exists. In addition, in the descriptions of embodiments of this application, "a plurality of" means two or more than two.

The following terms "first" and "second" are merely used for description, and shall not be understood as an indication or implication of relative importance or implicit indication of a quantity of indicated technical features. Therefore, a feature limited by "first" or "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language such as Java or an extensible markup language (extensible markup language, XML). Interface source code is parsed and rendered on an electronic device, and is finally presented as content that can be identified by the user. The user interface is usually represented in a form of graphical user interface (graphical user interface, GUI), and is a user interface that is related to a computer operation and that is displayed in a graphic manner. The user interface may be a visual interface element, for example, a text, an icon, a button, a menu, a tab, a text box, a dialog box, a status bar, a navigation bar, or a Widget that is displayed on a display of the electronic device.

The following first describes some technical terms in embodiments of this application.

### 1. Frame segmentation

Usually, a voice has a time-varying property. That is, a characteristic of a voice signal changes with time, which is a non-stationary random process. However, on the other hand, although the voice signal has the time-varying property, the characteristic of the voice signal may be considered steady in a short time. Therefore, the voice signal may be divided into short-time frames, and may be considered as a steady-state signal in each frame. For example, a frame length may be usually 25 milliseconds. To avoid an excessive change between two adjacent frames, the frames may overlap each other to compensate for the change. A time difference between start positions of two adjacent frames is called a frame shift. Usually, the frame shift may be half of the frame length, or may be fixed to 10 milliseconds.

### 2. Windowing

Usually, after frame segmentation is performed on a voice signal, a windowing operation needs to be performed on the voice signal to reduce spectrum leakage. The windowing operation is to multiply the voice signal by a window function. Common window functions include a rectangular window, a Hamming window, and a Hanning window. Different window functions may be selected based on different situations.

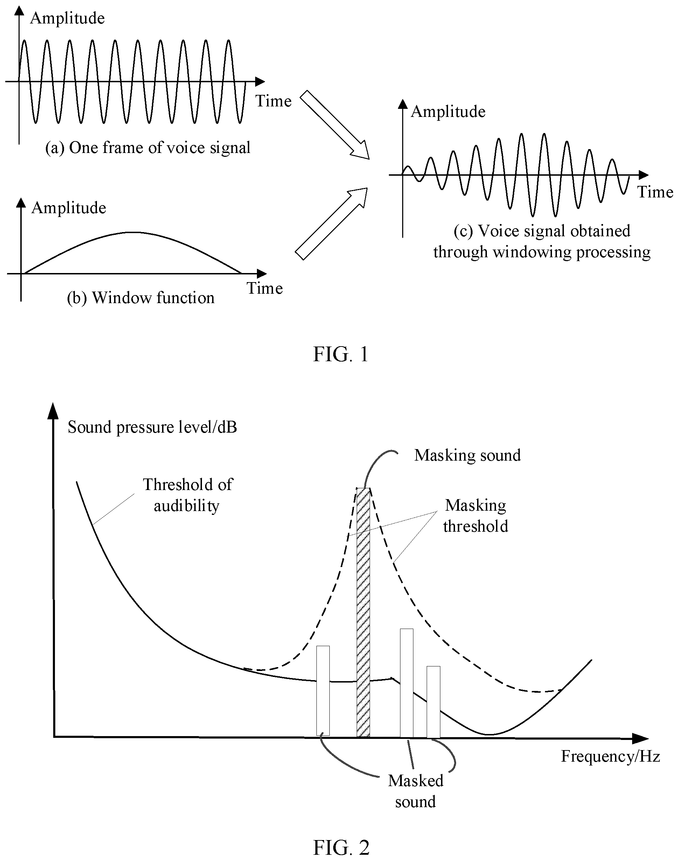

FIG. 1 shows an example of a diagram of windowing one frame of voice signal. As shown in FIG. 1, a horizontal axis of coordinates in FIG. 1 represents time, and a vertical axis represents amplitude. (a) in FIG. 1 is a waveform diagram of the voice signal existing before windowing. (b) in FIG. 1 is a waveform diagram of a window function. (c) in FIG. 1 is a waveform diagram of a voice signal obtained through windowing. It can be learned that, after windowing processing is performed on the frame of voice signal, amplitude of the frame of voice signal gradually changes to 0 at two ends, and two end parts of the frame of voice signal are weakened after windowing.

### 3. Masking effect

A phenomenon that an auditory feeling of a weak sound (masked sound) is affected by another strong sound (masking sound) is called the "masking effect" of a human ear. The masking effect is another important physiological feature of the human ear. If there are two types of sound signals in a narrow frequency band, when intensity of one type of sound signal is greater than that of the other type of sound signal, an auditory threshold of the human ear is increased, and the human ear may hear a high-volume sound signal, but cannot hear a low-volume sound signal at a nearby frequency of the high-volume sound signal. It seems that the low-volume signal is masked by the high-volume signal. The auditory threshold (namely, a threshold of audibility) of the human ear is a lowest sound pressure level at which a sound can be sensed by the human ear.

FIG. 2 is a diagram of masking effect according to an embodiment of this application. As shown in FIG. 2, a horizontal axis of a coordinate system represents a frequency in a unit of Hertz (Hz), and a vertical axis represents a sound pressure level in a unit of decibel (dB). In FIG. 2, a solid line represents a threshold of audibility, a rectangle filled with oblique lines represents a masking sound, a dashed line represents a masking threshold, and blank rectangles represent masked sounds. When there is no masking sound, if a sound pressure level of a sound is greater than the threshold of audibility, the sound may be heard by a human ear. It can be learned from FIG. 2 that sound pressure levels of the masked sounds are all greater than the threshold of audibility. Therefore, when there is no masking sound, the human ear may hear the masked sound. After the masking sound appears, a sound pressure level of a sound at a nearby frequency of the masking sound needs to be greater than the masking threshold, so that the sound can be heard by the human ear. It can be learned from FIG. 2 that the sound pressure levels of the masked sounds are all less than the masking threshold. Therefore, after the masking sound appears, the masked sound that the human ear has heard becomes inaudible. In this case, provided that a sound pressure level of a sound in the masked sounds is greater than the masking threshold, for example, in FIG. 2, provided that a sound of at least one masked sound in the three masked sounds is greater than the masking threshold, the human ear can hear the masked sound.

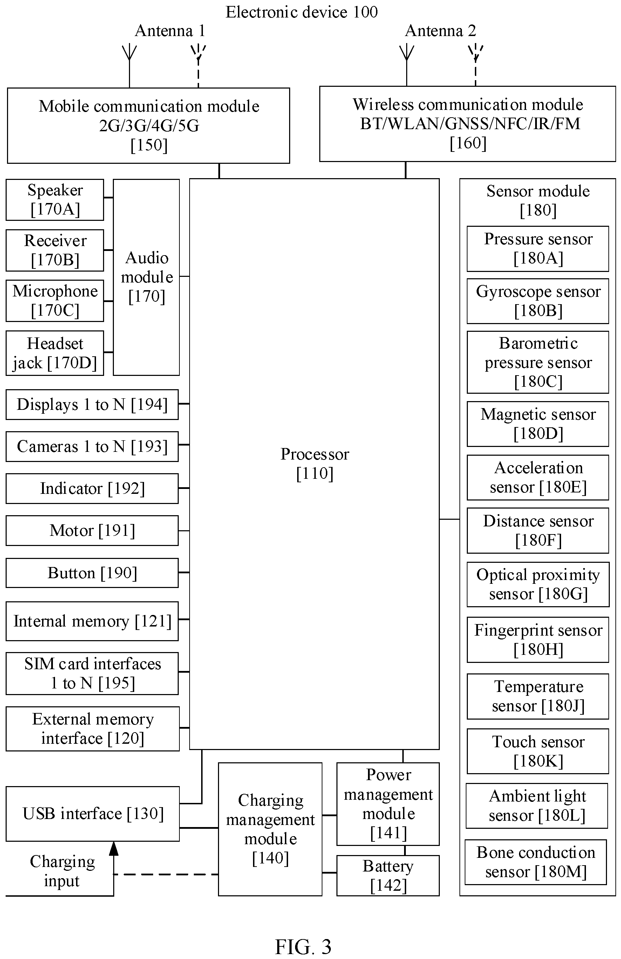

FIG. 3 is a diagram of a hardware structure of an electronic device 100.

The electronic device 100 is used as an example below to describe embodiments in detail. It should be understood that the electronic device 100 may include more or fewer components than those shown in the figure, two or more components may be combined, or there may be different component configurations. The components shown in the figure may be implemented in hardware, software, or a combination of hardware and software including one or more signal processing and/or application-specific integrated circuits.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of this application does not constitute a specific limitation on the electronic device 100. In some other embodiments of this application, the electronic device 100 may include more or fewer components than those shown in the figure, or some components may be combined, or some components may be split, or there may be a different component arrangement. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a nerve center and a command center of the electronic device 100. The controller may generate an operation control signal based on instruction operation code and a time sequence signal, to complete control of instruction reading and instruction execution.

The memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 180K, a charger, a flashlight, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transfer an audio signal to the wireless communication module 160 through the I2S interface, to implement a function of answering a call through a Bluetooth headset.

The PCM interface may also be configured to: perform audio communication, and sample, quantize, and encode an analog signal. In some embodiments, the audio module 170 may be coupled to the wireless communication module 160 through the PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be configured to perform audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transfer an audio signal to the wireless communication module 160 through the UART interface, to implement a function of playing music through a Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 communicates with the camera 193 through the CSI, to implement a photographing function of the electronic device 100. The processor 110 communicates with the display 194 through the DSI, to implement a display function of the electronic device 100.

The GPIO interface may be configured by using software. The GPIO interface may be configured as a control signal, or may be configured as a data signal. In some embodiments, the GPIO interface may be configured to connect the processor 110 to the camera 193, the display 194, the wireless communication module 160, the audio module 170, the sensor module 180, and the like. The GPIO interface may alternatively be configured as an I2C interface, an I2S interface, a UART interface, an MIPI interface, or the like.

The SIM card interface may be configured to communicate with the SIM card interface 195, to implement a function of transmitting data to a SIM card or reading data in a SIM card.

The USB interface 130 is an interface that conforms to a USB standard specification, and may be specifically a mini USB interface, a micro USB interface, a USB Type-C interface, or the like. The USB interface 130 may be configured to connect to the charger to charge the electronic device 100, or may be configured to transmit data between the electronic device 100 and a peripheral device, or may be configured to connect to a headset for playing audio through the headset. The interface may be alternatively configured to connect to another electronic device such as an AR device.

It may be understood that, an interface connection relationship between the modules shown in this embodiment of this application is merely an example for description, and does not constitute a limitation on the structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive a charging input from the charger. The charger may be a wireless charger or a wired charger.

The power management module 141 is configured to connect to the battery 142, the charging management module 140, and the processor 110. The power management module 141 receives an input of the battery 142 and/or the charging management module 140, to supply power to the processor 110, the internal memory 121, an external memory, the display 194, the camera 193, the wireless communication module 160, and the like.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 can provide a wireless communication solution that is applied to the electronic device 100 and that includes 2G/3G/4G/5G, or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit a processed electromagnetic wave to the modem processor for demodulation. The mobile communication module 150 may further amplify a signal modulated by the modem processor, and convert an amplified signal into an electromagnetic wave for radiation through the antenna 1. In some embodiments, at least some functional modules of the mobile communication module 150 may be disposed in the processor 110. In some embodiments, at least some functional modules of the mobile communication module 150 and at least some modules of the processor 110 may be disposed in a same component.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (not limited to the speaker 170A, the receiver 170B, and the like), and displays an image or a video through the display 194. In some embodiments, the modem processor may be an independent component. In some other embodiments, the modem processor may be independent of the processor 110, and is disposed in a same component as the mobile communication module 150 or another functional module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 100 may implement a display function through the GPU, the display 194, the application processor, and the like. The GPU is a microprocessor for image processing, and is connected to the display 194 and the application processor. The GPU is configured to perform mathematical and geometric computation for graphic rendering. The processor 110 may include one or more GPUs that execute program instructions to generate or change display information.

The display 194 is configured to display an image, a video, or the like. The display 194 includes a display panel. The display panel may use a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), and the like. In some embodiments, the electronic device 100 may include one or N displays 194, where N is a positive integer greater than 1.

The electronic device 100 may implement a photographing function through the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera via a lens. An optical signal is converted into an electrical signal, and the photosensitive element of the camera transmits the electrical signal to the ISP for processing, to convert the electrical signal into a visible image. The ISP may further perform algorithm optimization on noise, luminance, and complexion of the image. The ISP may further optimize parameters such as exposure and a color temperature of a photographing scene. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts the optical signal into the electrical signal, and then transmits the electrical signal to the ISP to convert the electrical signal into a digital image signal. The ISP outputs the digital image signal to the DSP for processing. The DSP converts the digital image signal into an image signal in a standard format, for example, RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 performs frequency selection, the digital signal processor is configured to perform Fourier transform and the like on frequency energy.

The video codec is configured to compress or decompress a digital video. The electronic device 100 may support one or more types of video codecs. Therefore, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor. The NPU quickly processes input information with reference to a structure of a biological neural network, for example, a transfer mode between human brain neurons, and may further continuously perform self-learning. The NPU may implement applications such as intelligent cognition of the electronic device 100, for example, image recognition, facial recognition, voice recognition, and text understanding.

The external memory interface 120 may be configured to connect to an external storage card, for example, a micro SD card, to extend a storage capability of the electronic device 100. The external memory card communicates with the processor 110 through the external memory interface 120, to implement a data storage function. For example, files such as music and videos are stored in the external memory card.

The internal memory 121 may be configured to store computer-executable program code. The executable program code includes instructions. The processor 110 runs the instructions stored in the internal memory 121, to perform various function applications and data processing of the electronic device 100. The internal memory 121 may include a program storage area and a data storage area. The program storage area may store an operating system, an application required by at least one function (for example, a facial recognition function, a fingerprint recognition function, and a mobile payment function), and the like. The data storage area may store data (for example, facial information template data and a fingerprint information template) and the like created during use of the electronic device 100. In addition, the internal memory 121 may include a high-speed random access memory, or may include a nonvolatile memory, for example, at least one magnetic disk storage device, a flash memory, or a universal flash storage (universal flash storage, UFS).

The electronic device 100 may implement an audio function such as music playing or recording by using the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode an audio signal. In some embodiments, the audio module 170 may be disposed in the processor 110, or some functional modules of the audio module 170 are disposed in the processor 110.

In this embodiment of this application, the electronic device may play a sound signal by using a sound-emitting apparatus. The sound-emitting apparatus may be the following speaker 170A, or may be the following receiver 170B, or may be an external device connected to the electronic device, such as a headset or glasses, which is not limited herein.

The speaker 170A, also referred to as a "horn", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or voice information is received through the electronic device 100, the receiver 170B may be put close to a human ear to listen to a voice.

The microphone 170C, also referred to as a "mike" or a "mic", is configured to convert a sound signal into an electrical signal. When making a call or sending voice information, a user may make a sound by placing a mouth close to the microphone 170C, to input a sound signal to the microphone 170C. At least one microphone 170C may be disposed in the electronic device 100. In some other embodiments, two microphones 170C may be disposed in the electronic device 100, to collect a sound signal and further implement a noise reduction function. In some other embodiments, three, four, or more microphones 170C may alternatively be disposed in the electronic device 100, to collect a sound signal, reduce noise, further identify a sound source, implement a directional recording function, and the like.

The headset jack 170D is configured to connect to a wired headset. The headset jack 170D may be a USB interface 130, or may be a 3.5 mm open mobile terminal platform (open mobile terminal platform, OMTP) standard interface or cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change of the capacitance. When a touch operation is performed on the display 194, the electronic device 100 detects touch operation intensity by using the pressure sensor 180A. The electronic device 100 may calculate a touch position based on a detection signal of the pressure sensor 180A. In some embodiments, touch operations that are performed in a same touch position but have different touch operation intensities may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating an SMS message is performed.

The gyroscope sensor 180B may be configured to determine a motion posture of the electronic device 100. In some embodiments, angular velocities of the electronic device 100 around three axes (that is, an x axis, a y axis, and a z axis) may be determined by using the gyroscope sensor 180B. The gyroscope sensor 180B may be configured to implement image stabilization during photographing. For example, when the shutter is pressed, the gyroscope sensor 180B detects a shake angle of the electronic device 100, calculates, based on the angle, a distance that needs to be compensated for by a lens module, and allows the lens to cancel a shake of the electronic device 100 through reverse motion, to implement image stabilization. The gyroscope sensor 180B may be further used in a navigation scenario and a motion-sensing game scenario.

The barometric pressure sensor 180C is configured to measure barometric pressure. In some embodiments, the electronic device 100 calculates an altitude based on a barometric pressure value measured by the barometric pressure sensor 180C to assist in positioning and navigation.

The magnetic sensor 180D includes a Hall sensor. The electronic device 100 may detect opening and closing of a leather case of a flip cover by using the magnetic sensor 180D. In some embodiments, when the electronic device 100 is a flip phone, the electronic device 100 may detect opening and closing of the flip cover by using the magnetic sensor 180D. Further, a feature such as automatic unlocking of the flip cover is set based on a detected opening or closing state of the leather case or a detected opening or closing state of the flip cover.

The acceleration sensor 180E may detect magnitudes of accelerations of the electronic device 100 in various directions (usually on three axes). When the electronic device 100 is still, a magnitude and a direction of gravity may be detected. The acceleration sensor 180E may be further configured to identify a posture of the electronic device, and is used in an application such as switching between a landscape mode and a portrait mode or a pedometer.

The distance sensor 180F is configured to measure a distance. The electronic device 100 may measure a distance through infrared light or a laser. In some embodiments, in a photographing scenario, the electronic device 100 may measure a distance by using the distance sensor 180F, to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, the electronic device 100 may determine that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically perform screen-off for power saving. The optical proximity sensor 180G may also be used in a smart cover mode or a pocket mode to automatically perform screen unlocking or locking.

The ambient light sensor 180L is configured to sense ambient light luminance. The electronic device 100 may adaptively adjust luminance of the display 194 based on the sensed ambient light luminance. The ambient light sensor 180L may also be configured to automatically adjust white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may implement fingerprint unlock, application access lock, fingerprint photographing, fingerprint-based call answering, and the like by using a feature of the collected fingerprint.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is lower than another threshold, the electronic device 100 heats the battery 142 to avoid abnormal shutdown of the electronic device 100 caused due to the low temperature. In some still other embodiments, when the temperature is lower than still another threshold, the electronic device 100 boosts an output voltage of the battery 142 to avoid abnormal shutdown caused by the low temperature.

The touch sensor 180K is also referred to as a "touch panel". The touch sensor 180K may be disposed on the display 194, and the touch sensor 180K and the display 194 constitute a touchscreen, which is also referred to as a "touchscreen". The touch sensor 180K is configured to detect a touch operation performed on or near the touch sensor. The touch sensor may transfer the detected touch operation to the application processor to determine a type of a touch event. A visual output related to the touch operation may be provided by using the display 194. In some other embodiments, the touch sensor 180K may alternatively be disposed on a surface of the electronic device 100 at a position different from that of the display 194.

The button 190 includes a power button, a volume button, or the like. The button 190 may be a mechanical button, or may be a touch button. The electronic device 100 may receive a button input, and generate a button signal input related to user settings and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. The touch vibration feedback effect may be further customized.

The indicator 192 may be an indicator light, and may be configured to indicate a charging status and a power change, or may be configured to synthesize requests, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may also be compatible with different types of SIM cards. The SIM card interface 195 may also be compatible with the external memory card. The electronic device 100 interacts with a network by using a SIM card, to implement functions such as conversation and data communication.

In this embodiment of this application, the electronic device 100 may perform, by using the processor 110, a volume adjustment method provided in embodiments of this application.

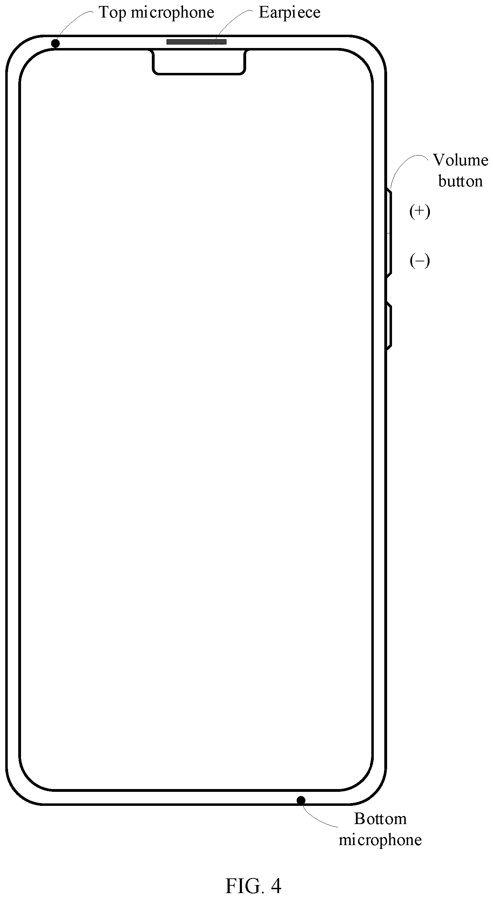

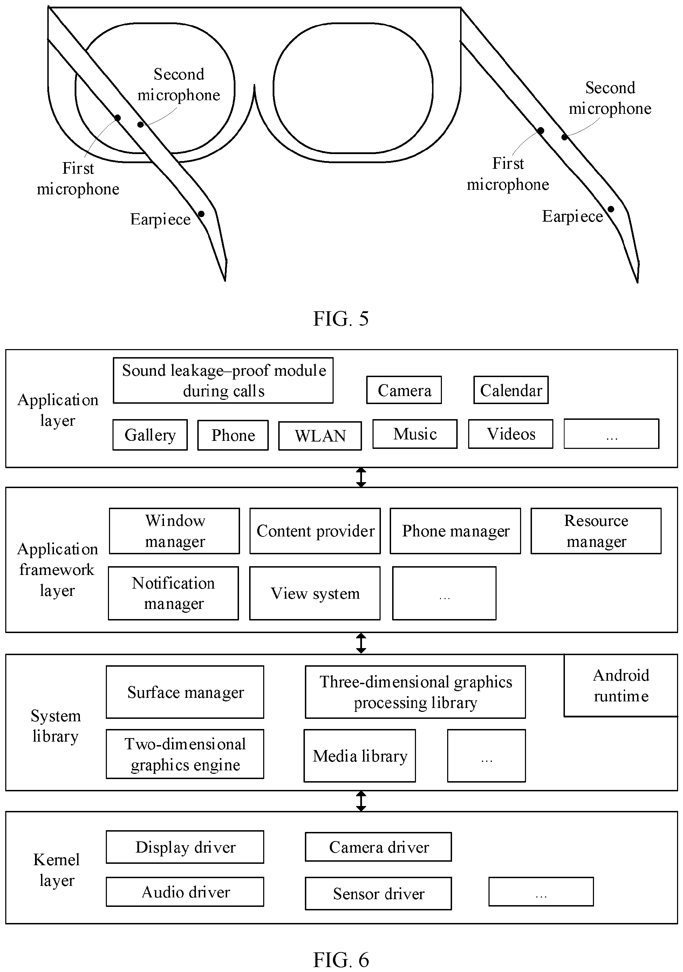

In this embodiment of this application, the electronic device 100 may include two microphones. One microphone is configured to obtain a noise signal, and the other microphone is configured to obtain a downlink signal. For example, the electronic device 100 may be a mobile phone shown in FIG. 4 or glasses shown in FIG. 5. The downlink signal is audio played by the electronic device by using the sound-emitting apparatus. For example, during a call, the downlink signal is a voice, of the other party in the call, played by the electronic device by using the earpiece. For another example, when a song is listened to, the downlink signal is a song played by the electronic device.

FIG. 4 shows an example of a hardware form of a mobile phone. The mobile phone may play a downlink signal in a plurality of play forms, for example, by using an earpiece and a headset. Different play forms may correspond to different preset models or volume adjustment methods. The following uses the earpiece as an example to describe a process in which the mobile phone collects the downlink signal and a noise signal.

As shown in FIG. 4, one microphone of the mobile phone is located on one side of the earpiece, and the other microphone of the mobile phone is located on an opposite side of the earpiece. For ease of description, in this embodiment of this application, the microphone located on the side of the earpiece is referred to as a top microphone (briefly referred to as a top microphone), the microphone located on the opposite side of the earpiece is referred to as a bottom microphone (briefly referred to as a bottom microphone). It may be understood that the top microphone is located close to the earpiece, so that the downlink signal played by the earpiece can be better collected. Therefore, in some embodiments of this application, a signal collected by the top microphone may be used as the downlink signal, and a signal collected by the bottom microphone may be used as the noise signal.

It should be noted that, in some other embodiments of this application, the mobile phone may determine the downlink signal from signals collected by the top microphone. For example, the mobile phone identifies the noise signal from the signals collected by the top microphone and removes the noise signal, to obtain the downlink signal. For another example, the mobile phone compares the signals collected by the top microphone with signals collected by the bottom microphone, and uses a difference part between the signals collected by the top microphone and the signals collected by the bottom microphone as the downlink signal.

In addition, as shown in FIG. 4, the mobile phone may further include a volume button. A side marked with (+) is a volume up button of the volume button, and a side marked with (-) is a volume down button of the volume button. The volume button is configured to adjust a sound level of the earpiece or a speaker. For example, the mobile phone includes 10 sound levels. A higher level indicates a higher play volume. A user may determine a higher sound level by pressing the volume up button, so that the play volume increases.

FIG. 5 shows an example of a hardware form of glasses. As shown in FIG. 5, two microphones and earpieces may be respectively disposed on two glasses temples of the glasses. As shown in FIG. 5, because a second microphone is close to the earpiece, the second microphone is configured to obtain a downlink signal, and a first microphone is configured to obtain a noise signal.

It should be noted that, in some embodiments of this application, a signal collected by the second microphone may be used as the downlink signal, and a signal collected by the first microphone may be used as the noise signal. In some other embodiments of this application, the downlink signal may be determined from signals collected by the second microphone. For example, the noise signal is identified from the signals collected by the second microphone and the noise signal is removed, to obtain the downlink signal. For another example, the signals collected by the second microphone are compared with signals collected by the bottom microphone, and a difference part between the signals collected by the second microphone and the signals collected by the bottom microphone is used as the downlink signal.

FIG. 6 is a block diagram of a software structure of an electronic device 100 according to an embodiment of this application.

In a layered architecture, software is divided into several layers, and each layer has a clear role and task. The layers communicate with each other through a software interface. In some embodiments, a system is divided into four layers from top to bottom: an application layer, an application framework layer, runtime (Runtime) and a system library, and a kernel layer.

The application layer may include a series of application packages.

As shown in FIG. 6, the application layer further includes a sound leakage-proof module during calls. The application packages may include applications (or referred to as applications) such as Camera, Gallery, Calendar, Phone, Map, Navigation, WLAN, Bluetooth, Music, Videos, and Messages.

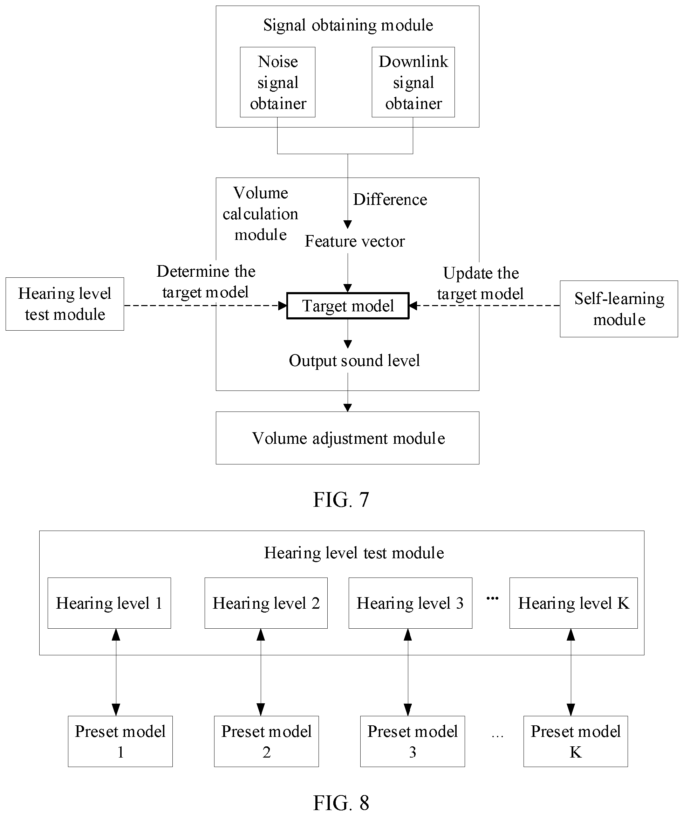

The sound leakage-proof module during calls is configured to adjust a play volume of a downlink signal in a call process. The sound leakage-proof module during calls may specifically include a hearing level test model, an audio calculation module, a self-learning module, and the like shown in FIG. 7. For specific content of the hearing level test model, the audio calculation module, the self-learning module, and the like, refer to related content in the following. Details are not described herein again.

The application framework layer provides an application programming interface (application programming interface, API) and a programming framework for an application at the application layer. The application framework layer includes some predefined functions.

As shown in FIG. 6, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like.

The content provider is configured to: store and obtain data, and enable the data to be accessed by an application. The data may include a video, an image, audio, calls that are made and answered, a browsing history and bookmarks, a phone book, and the like.

The view system includes visual controls, such as a control for displaying a text and a control for displaying a picture. The view system may be configured to construct an application. A display interface may include one or more views. For example, a display interface including an SMS message notification icon may include a view for displaying text and a view for displaying a picture.

The phone manager is configured to provide a communication function for the electronic device 100, for example, management of a call status (including answering, declining, or the like).

The resource manager provides various resources such as a localized character string, an icon, a picture, a layout file, and a video file for an application.

The notification manager enables an application to display notification information in the status bar, and may be configured to convey a notification-type message. The displayed notification information may automatically disappear after a short pause without user interaction. For example, the notification manager is configured to notify download completion, provide a message notification, and the like. The notification manager may alternatively be a notification that appears in a top status bar of the system in a form of graph or scroll bar text, for example, a notification of an application running in the background, or a notification that appears on a screen in a form of dialog interface. For example, text information is displayed in the status bar, a prompt tone is made, the electronic device vibrates, or an indicator light flashes.

The runtime (Runtime) includes a kernel library and a virtual machine. The runtime is responsible for scheduling and management of the system.

The kernel library includes two parts: a performance function that needs to be invoked in a programming language (for example, a Java language), and a system kernel library.

The application layer and the application framework layer run on the virtual machine. The virtual machine executes programming files (for example, Java files) of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (Media Libraries), a three-dimensional graphics processing library (for example, OpenGL ES), and a two-dimensional graphics engine (for example, SGL).

The surface manager is configured to manage a display subsystem and provide fusion of two-dimensional (two-dimensional, 2D) and three-dimensional (three-dimensional, 3D) layers for a plurality of applications.

The media library supports playback and recording in a plurality of commonly used audio and video formats, static image files, and the like. The media library may support a plurality of audio and video encoding formats, for example, MPEG-4, H.264, MP3, AAC, AMR, JPG, and PNG.

The three-dimensional graphics processing library is configured to implement 3D graphics drawing, image rendering, synthesis, layer processing, and the like.

The 2D graphics engine is a drawing engine for 2D drawing.

The kernel layer is a layer between hardware and software. The kernel layer includes at least a display driver, a camera driver, an audio driver, a sensor driver, and a virtual card driver.

The following describes an example of a working process of the software and the hardware of the electronic device 100 with reference to a capturing and photographing scenario.

When the touch sensor 180K receives a touch operation, a corresponding hardware interruption is sent to the kernel layer. The kernel layer processes the touch operation into an original input event (including information such as touch coordinates and a timestamp of the touch operation). The original input event is stored at the kernel layer. The application framework layer obtains the original input event from the kernel layer, and identifies a control corresponding to the input event. For example, the touch operation is a touch tap operation, and a control corresponding to the tap operation is a control of a camera application icon. A camera application invokes an interface of the application framework layer to start the camera application, then invokes the kernel layer to start the camera driver, and captures a static image or a video through the camera 193.

First, with reference to FIG. 7, an overall procedure of a volume adjustment method provided in some embodiments of this application is described.

As shown in FIG. 7, an electronic device may include a signal obtaining module, a hearing level test module, a volume calculation module, a self-learning module, and a volume adjustment module. The signal obtaining module may include a noise signal obtainer and a downlink signal obtainer. For example, the noise signal obtainer and the downlink signal obtainer may be microphones of the electronic device 100. For another example, the downlink signal obtainer may be the top microphone of the mobile phone shown in FIG. 4, and the noise signal obtainer may be the bottom microphone of the mobile phone shown in FIG. 4. For another example, the downlink signal obtainer may be the first microphone of the glasses shown in FIG. 5, and the noise signal obtainer may be the second microphone of the glasses shown in FIG. 5.

The volume calculation module may determine a feature vector based on a difference between a noise signal obtained by the noise signal obtainer and a downlink signal obtained by the downlink signal obtainer. The feature vector may include energy differences between frequency band-specific signals of the noise signal and frequency band-specific signals of the downlink signal. If a decibel value of a signal in any frequency band in the frequency band-specific signals of the downlink signal is greater than a masking threshold in masking effect, the downlink signal may be heard by a user. The feature vector is input into a target model, so that an output sound level may be obtained. Sound levels of the electronic device include the output sound level. The target model may be determined based on an application corresponding to playing audio, a play manner of playing the audio, and the user. For details, refer to related content in the following.

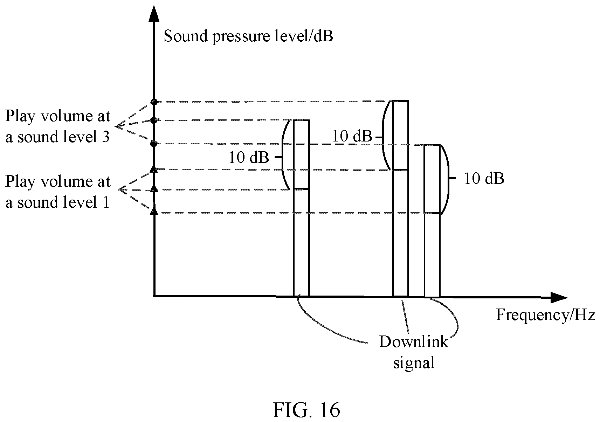

The volume adjustment module may be configured by the electronic device to adjust a play volume of the downlink signal based on the output sound level. The sound level is used to adjust a play volume of the audio. The electronic device may play the audio at different sound levels. Play volumes of the audio vary when the electronic device plays the audio at the different sound levels. For details, refer to related descriptions in FIG. 16.

The target model may be a preset model determined by the hearing level test module from several preset models based on a hearing level of the user. Alternatively, the target model may be a preset model corresponding to the application corresponding to playing the audio. The hearing level test module is configured to test the hearing level of the user, and a preset model corresponding to the hearing level of the user is the target model. The self-learning module is configured to train the target model based on use data of the user, to update the target model, so that an output result of the target model better conforms to a use habit of the user.

The following describes a principle of the preset model.

FIG. 8 shows an example of correspondences between K preset models and hearing levels, where K is a positive integer. As shown in FIG. 8, a hearing level test module may classify hearing levels of users into K hearing levels, which are a hearing level 1, and a hearing level 2, to a hearing level K. Each hearing level corresponds to one preset model. For example, a preset model corresponding to the hearing level 1 is a preset model 1, a preset model corresponding to the hearing level 2 is a preset model 2, and a preset model corresponding to the hearing level K is a preset model K. For example, if the hearing level 1 is a hearing level of an age group 1, the preset model 1 is a model that may output a hearing requirement that satisfies the age group 1. If the hearing level 2 is a hearing level of an age group 2, the preset model 2 is a model that may output a hearing requirement that satisfies the age group 2.

It should be noted that, because each preset model may be obtained through training based on a user training set corresponding to a hearing level, a preset module may meet a hearing requirement of a user corresponding to the preset module.

The user training set includes a plurality of feature vectors and a target sound level corresponding to each feature vector, and each feature vector is obtained based on a difference between an uplink signal and a noise signal. The target sound level may be a comfortable sound level for the user or a lowest sound level that the user is able to clearly hear. For example, if an electronic device plays audio at a first sound level, and the user considers that a play volume of the audio is comfortable, the first sound level is the comfortable sound level for the user. If the electronic device plays the audio at the first sound level, and the user considers that the play volume of the audio is a lowest volume at which the user is able to clearly hear, the first sound level is the lowest sound level that the user is able to clearly hear.

It may be understood that if the target sound level is the comfortable sound level, an output sound level of a preset model trained based on the target sound level is the comfortable sound level for the user. If the target sound level is the lowest sound level that the user is able to clearly hear, an output sound level of the preset model trained based on the target sound level is the lowest sound level that the user is able to clearly hear or a low sound level.

The following uses the comfortable sound level as an example to describe a process of obtaining the user training set.

First, comfortable sound levels of several people in different scenarios and downlink signals and noise signals corresponding to the volumes may be collected. Scenario dimensions described herein include a size and a type of ambient noise, playing different sound sources, and the like. Further, a feature vector corresponding to each comfortable sound level is calculated based on the downlink signal and the noise signal. In this case, one comfortable sound level and a feature vector corresponding to the comfortable sound level may be used as one piece of training data of the user training set. The comfortable sound level is the foregoing target sound level. In this way, the user training set is obtained. It should be noted that, to avoid short-time volume fluctuation, smoothing processing may be further performed on comfortable sound levels of two consecutive frames of voices.

It should be noted that user training sets corresponding to different hearing levels may be obtained based on user data of the different hearing levels. For example, if the hearing level of the age group 1 is the hearing level 1, a user training set corresponding to the hearing level 1 is obtained based on a comfortable sound level for the age group 1 and a downlink signal and a noise signal that correspond to the volume. For another example, if the hearing level of the age group 2 is the hearing level 2, a user training set corresponding to the hearing level 2 is obtained based on a comfortable sound level of the age group 2 and a downlink signal and a noise signal that correspond to the volume.

The following uses the preset model 1 as an example to describe a training process of the preset model.

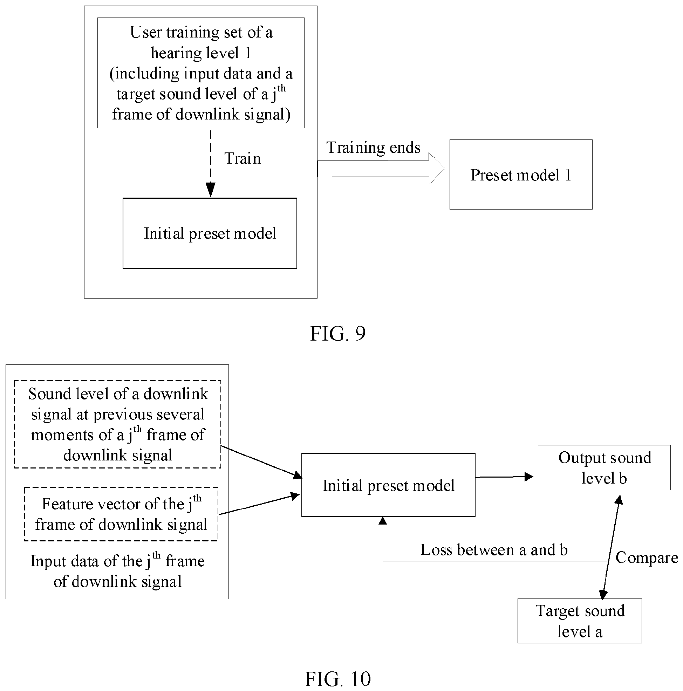

Refer to FIG. 9. The electronic device may first obtain the user training set of the hearing level 1. The user training set may include a plurality of pieces of training data. For example, the plurality of pieces of training data include a j^{th} piece of training data, and the j^{th} piece of training data includes input data and a target sound level of a j^{th} frame of downlink signal. The electronic device may train an initial preset model based on each piece of training data, and end the training when a loss meets a preset condition, to obtain the preset model 1.

FIG. 10 uses a j^{th} piece of training data as an example to describe a training process. The j^{th} piece of training data includes input data and a target sound level a of a j^{th} frame of downlink signal. The input data of the j^{th} frame of downlink signal may include a sound level of an earpiece at previous several moments of the j^{th} frame of downlink signal and a feature vector of the j^{th} frame of downlink signal. The feature vector of the j^{th} frame of downlink signal is obtained based on a difference between the j^{th} frame of downlink signal and a j^{th} frame of noise signal. The electronic device inputs the j^{th} piece of training data into an initial preset model, to obtain an output sound level b. The electronic device compares the target sound level a with the output sound level b to obtain a loss between the target sound level a and the output sound level b, and inputs a loss feedback value into the initial preset model.

It should be noted that, in some other embodiments, the input data of the j^{th} frame of downlink signal may not include the sound level of the earpiece at the previous several moments of the j^{th} frame of downlink signal. In this embodiment of this application, the input data includes the sound level of the earpiece at the previous several moments, to smoothly change a volume, and avoid a case in which the volume suddenly increases excessively or decreases excessively.

It may be understood that the foregoing preset model is only modeling of an average value of big data of volume use habits of users. In the volume adjustment method provided in embodiments of this application, considering that there is a large difference between hearing levels and hearing habits of actual user individuals, the foregoing preset model is adaptively learned and updated based on a usage history of each user, to achieve effect that more use of the users indicates a more accurate volume adjustment, and meet a personalized requirement of each user.

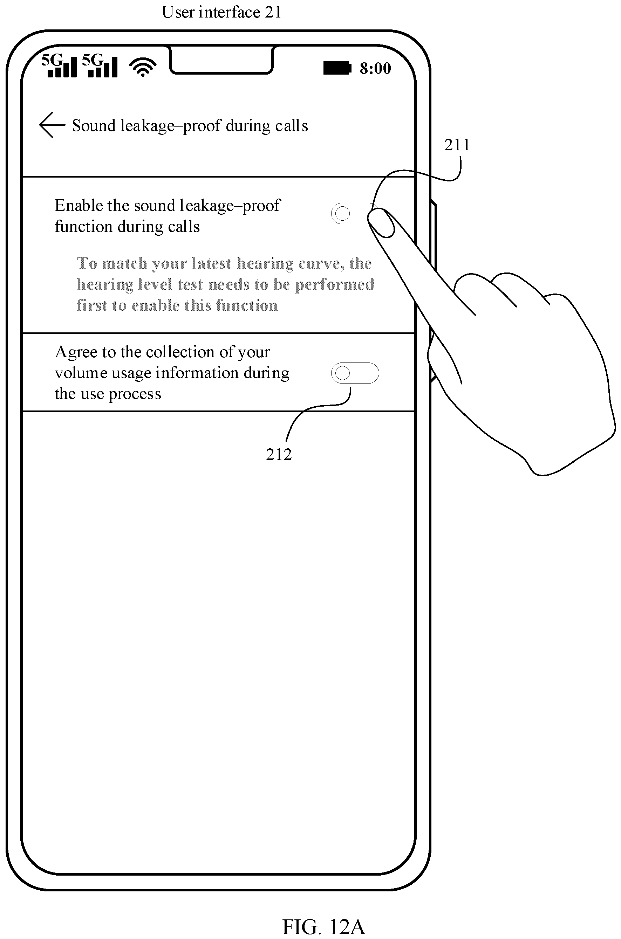

Specifically, in an actual use process of the user, the electronic device may record a feature vector each time the user manually adjusts the volume, a sound level obtained through the adjustment, and a sound level at previous several moments of the adjustment. The feature vector is a difference between a downlink signal and a noise signal that are collected when the user manually adjusts the volume. The electronic device may use the feature vector, the sound level obtained through the adjustment, and the sound level at the previous several moments of the adjustment as one piece of training data, and store the piece of training data in a self-learning training set. The feature vector and the sound level at the previous several moments of the adjustment are input data, and the sound level obtained through the adjustment is a target sound level. Further, when a quantity of pieces of training data in the self-learning training set satisfies a preset quantity, the electronic device may train a target model based on the self-learning training set at a proper occasion (for example, at night). For example, when the user taps an agree control 212 in a user interface 21 shown in FIG. 12A, the electronic device detects, in each call process of the user, a volume adjustment operation of the user in the call process. When the user performs the volume adjustment operation, a feature vector obtained based on a downlink signal and a noise signal at this time, a sound level obtained through the user adjustment, and a sound level at previous several moments of the adjustment are saved as one piece of training data.

In another implementation, the electronic device may alternatively send the target model and the self-learning training set to a server. The server trains the target model by using the self-learning training set, and then the server sends a trained target model to the electronic device.

In some embodiments, different applications on the electronic device may correspond to different preset models. For example, preset models corresponding to an application 1 are a model 1, a model 2, and a model 3, and preset models corresponding to an application 2 are a model 4, a model 5, and a model 6. Further, the electronic device may determine, from preset models corresponding to an application and based on a hearing level of the user, a target model corresponding to the application, and perform a volume adjustment, based on the target model, on audio played by the application. The model 1, the model 2, and the model 3 are obtained through training based on user data obtained when users at different hearing levels use the application 1, and the model 4, the model 5, and the model 6 are obtained through training based on user data obtained when the users at the different hearing levels use the application 2. For a training process, refer to the foregoing related descriptions. Details are not described herein again.

The application 1 may be a call-type application, and the application has a call function. The user may perform video communication or voice communication with another user through this software. For example, the application 1 may be a call application embedded in a mobile phone system or MeeTime. The application 2 may be a music-type application, and the application has an audio playing function. For example, the application 2 may be audio software embedded in the mobile phone system, HUAWEI Music, or HUAWEI Video. It may be understood that, usually, the user has a high confidentiality requirement on the call-type application, and adjusts a volume of a downlink signal of the call-type application to a small value. Therefore, in a same environment, an output sound level of a target model of the call-type application is lower than an output sound level of the music-type application.

In an implementation, a hearing test result in the electronic device may be applied to a plurality of applications. That is, one hearing level may correspond to preset models of different applications. For example, when determining that a hearing level of a user A is a hearing level 1, the electronic device may determine that a target model corresponding to the application 1 is the model 1, and a target model corresponding to the application 2 is the model 3.

In another implementation, the electronic device may be configured with different hearing level tests for the different applications. For example, a hearing test corresponding to the application 1 is a hearing test 1, and a hearing test corresponding to the application 2 is a hearing test 2. It is assumed that a test result of the user A in the hearing test 1 is a hearing level 3, and a test result of the user A in the hearing test 2 is a hearing level 2, where a target model corresponding to the hearing level 3 is the model 3, and a target model corresponding to the hearing level 2 is the model 5. In this case, the target model corresponding to the application 1 is the model 3, and the target model corresponding to the application 2 is the model 5.

The volume adjustment method provided in embodiments of this application may be applied to a plurality of scenarios, for example, a call scenario, for example, the user answers a call through a mobile phone or glasses; or a song listening scenario, for example, the user wears glasses to listen to a song. This is not limited herein.

The following describes in detail the volume adjustment method provided in embodiments of this application by using a scenario in which the electronic device is a mobile phone and the user answers a call through the mobile phone as an example.

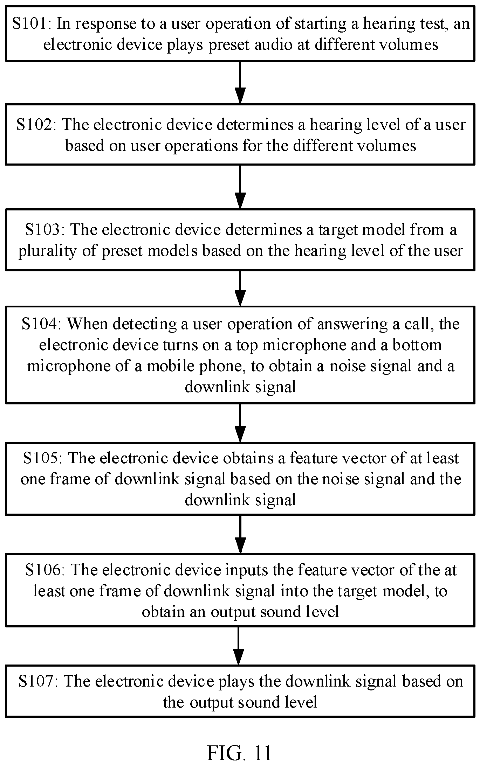

FIG. 11 is an overall schematic flowchart of a volume adjustment method according to this application. The method may include the following steps.

### Step S101: In response to a user operation of starting a hearing test, an electronic device plays preset audio at different volumes.

The volume may be a play volume or a sound level, and each volume corresponds to one preset hearing level. For example, the electronic device separately plays the preset audio at play volumes of 5 decibels, 15 decibels, 25 decibels, and 35 decibels. Each play volume corresponds to one hearing level. For another example, the electronic device plays the preset audio at different preset sound levels. For example, the electronic device includes 10 sound levels, the preset sound levels are five sound levels in the 10 sound levels, and five pieces of audio respectively correspond to five hearing levels. A higher sound level indicates higher loudness of audio played by an earpiece.

In some embodiments, system settings of the electronic device may include an option of a sound leakage-proof function during calls. Therefore, a user may enable the sound leakage-proof function during calls through a user operation on the option of the sound leakage-proof function during calls. Correspondingly, when the user operation is detected by the electronic device, the electronic device plays the preset audio at the different volumes in response to the user operation. The preset audio may be a piece of news, music, or the like. This is not limited herein.

In an implementation, the electronic device may play the preset audio of same content at volumes in ascending order. It should be noted that, if the electronic device plays the preset audio of the same content at volumes in descending order, because the user has heard the audio content clearly when the preset audio is played at a high volume, this method affects accuracy of determining, by the user, whether the audio content can be heard clearly at a low volume.

FIG. 12A to FIG. 12F are diagrams of some example interfaces in which a user performs a hearing test on an electronic device according to an embodiment of this application.

FIG. 12A shows an example of a user interface 21 in system settings of the electronic device. The user interface 21 includes a sound leakage-proof switch 211 and an agree control 212. The sound leakage-proof switch 211 is configured to enable the sound leakage-proof function during calls, and prompt information "To match your latest hearing curve, the hearing level test needs to be performed first to enable this function" is displayed below the sound leakage-proof switch 211. The agree control 212 is configured to obtain volume usage information of the user, where the volume usage information is used to train a target model.

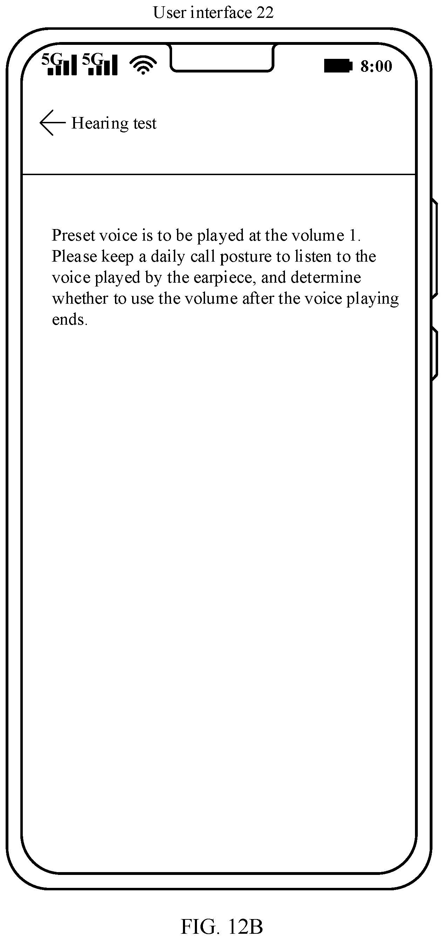

As shown in FIG. 12A, when a user operation performed by the user on the sound leakage-proof switch 211 is detected by the electronic device, the electronic device may display, in response to the user operation, a user interface 22 shown in FIG. 12B. The user interface 22 displays prompt information "Preset voice is to be played at the volume 1. Please keep a daily call posture to listen to the voice played by the earpiece, and determine whether to use the volume after the voice playing ends." The volume 1 may be a lowest volume. It may be understood that, after seeing the prompt information in the user interface 22, the user may place a mobile phone near an ear, and wait for the voice playing.

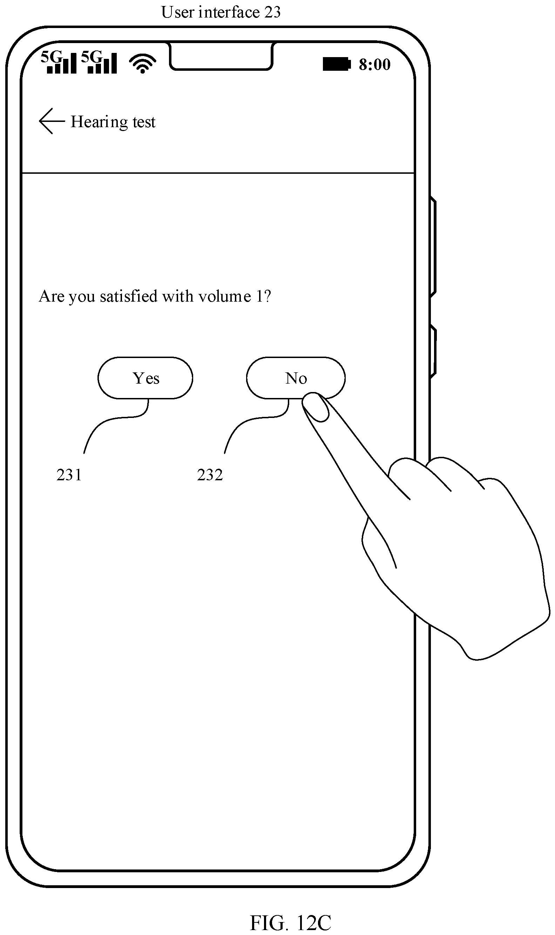

Further, after displaying the user interface 22 for several seconds, the electronic device may start to play the preset audio at the volume 1. After the playing ends, the electronic device may display a user interface 23 shown in FIG. 12C. The user interface 23 may include a satisfaction control 231 and a dissatisfaction control 232. The satisfaction control 231 is configured to indicate that the volume 1 is a satisfactory volume for the user, and the dissatisfaction control 232 is configured to indicate that the volume 1 is a dissatisfactory volume for the user. It may be understood that the user may view a mobile phone interface when the voice playing ends, and select a corresponding option based on user experience in a voice playing process when the mobile phone displays the user interface 23 shown in FIG. 12C.

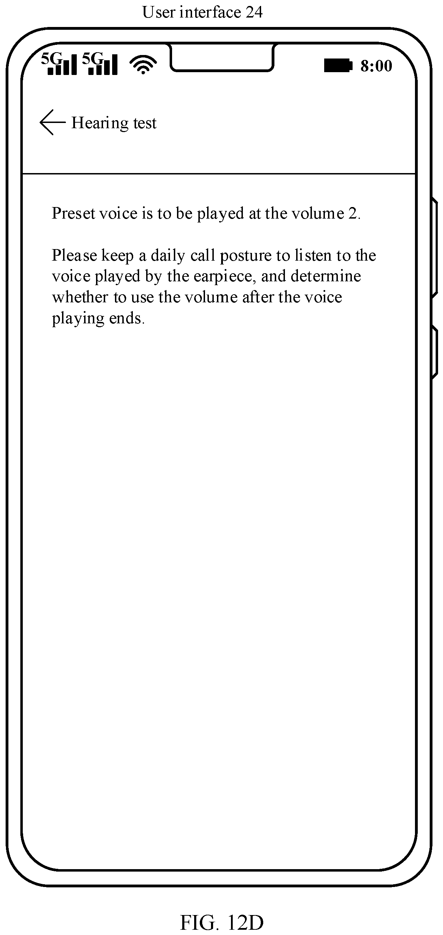

For example, if the user considers that the volume 1 is excessively low, the user may tap the dissatisfaction control 232. In this case, when a user operation performed by the user on the dissatisfaction control 232 is detected by the electronic device, the electronic device may display, in response to the user operation, a user interface 24 shown in FIG. 12D. The user interface 24 displays prompt information "Preset voice is to be played at the volume 2. Please keep a daily call posture to listen to the voice played by the earpiece, and determine whether to use the volume after the voice playing ends." The volume 2 may be a volume that is one level higher than the volume 1. For example, volumes of the electronic device are classified into five levels, and are the volume 1, the volume 2, a volume 3, a volume 4, and a volume 5 in ascending order.

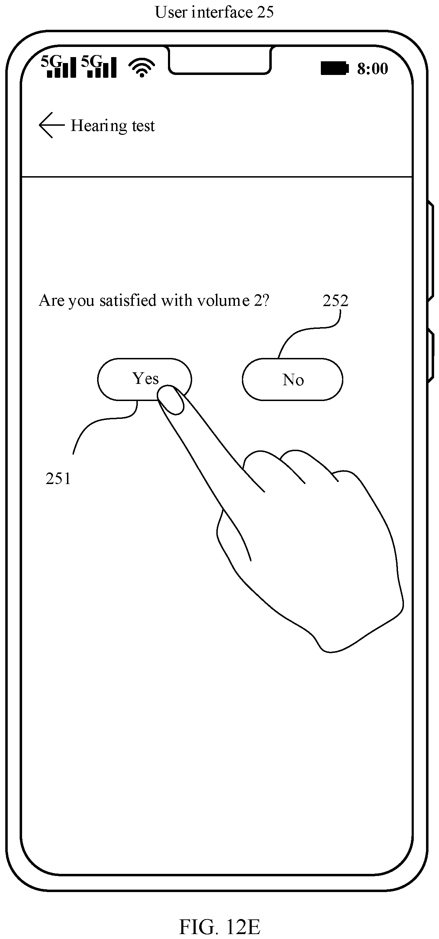

Further, after displaying the user interface 24 for several seconds, the electronic device may start to play the preset audio at the volume 2. After the playing ends, the electronic device may display a user interface 25 shown in FIG. 12E. The user interface 25 may include a satisfaction control 251 and a dissatisfaction control 252. The satisfaction control 251 is configured to indicate that the volume 2 is a satisfactory volume for the user, and the dissatisfaction control 252 is configured to indicate that the volume 2 is a dissatisfactory volume for the user.

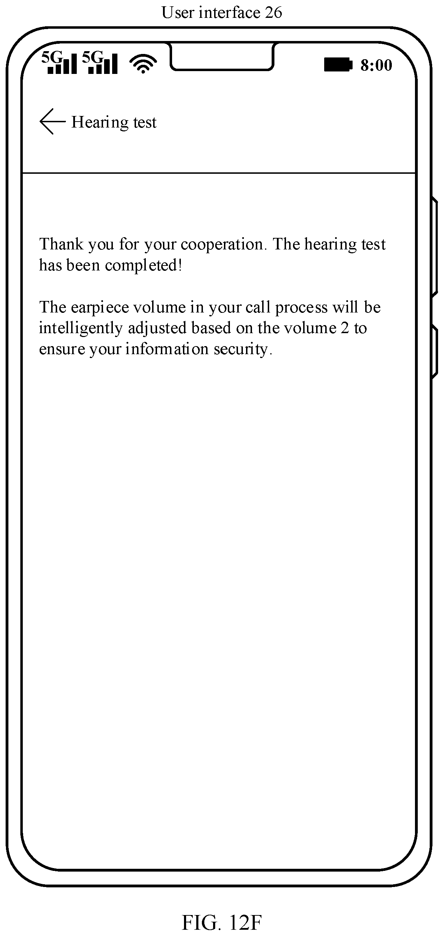

For example, if the user considers that the volume 2 meets a hearing requirement of the user, the user may tap the satisfaction control 251. In this case, when a user operation performed by the user on the satisfaction control 251 is detected by the electronic device, the electronic device displays, in response to the user operation, a user interface 26 shown in FIG. 12F. The user interface 26 displays prompt information "Thank you for your cooperation. The hearing test has been completed! The earpiece volume in your call process will be intelligently adjusted based on the volume 2 to ensure your information security."

In some embodiments, when a user operation on a dissatisfaction control is detected by the electronic device, the electronic device may play the preset audio at a next volume. For example, when the user operation performed by the user on the dissatisfaction control 232 of the volume 1 is detected by the electronic device, the electronic device plays the preset voice at the volume 2 to continue the hearing test. When a user operation on a satisfaction control is detected by the electronic device, the electronic device determines that a volume indicated by the satisfaction control is a satisfactory volume for the user, and the electronic device may end the hearing test. For example, when the user operation performed by the user on the satisfaction control 251 of the volume 2 is detected by the electronic device, the electronic device determines that the volume 2 is the satisfactory volume for the user, displays the user interface 26 shown in FIG. 12F, and does not perform the hearing test.

In some other embodiments, when the user operation on the satisfaction control is detected by the electronic device, the electronic device may continue to perform the hearing test, to complete tests for all the volumes. For example, all the volumes include the volume 1 to the volume 5. The volume 1 is the lowest volume, and the volume 5 is a highest volume. The electronic device may play the preset audio at all the volumes, and display a user interface similar to the user interface 23 shown in FIG. 12C for the user to perform selection for each volume. After detecting that the user completes selection of all the volumes, the electronic device ends the hearing test.

### Step S102: The electronic device determines a hearing level of the user based on user operations for the different volumes.

In some embodiments, in response to a satisfaction operation of the user for a volume, the electronic device determines a hearing level corresponding to the volume as the hearing level of the user. For example, as shown in FIG. 12A to FIG. 12F, when the user operation performed by the user on the satisfaction control 251 in the user interface 25 is detected by the electronic device, the electronic device determines the volume 2 as the satisfactory volume for the user. In this case, the electronic device may determine a hearing level 2 corresponding to the volume 2 as the hearing level of the user.

For example, if the volume 1 to the volume 5 are sequentially play volumes in ascending order, a user with good hearing may select the volume 1, and a user with poor hearing may select the volume 5. A hearing level corresponding to the volume 1 is a hearing level of a user with worst hearing, and a hearing level corresponding to the volume 5 may be a hearing level of a user with best hearing.

### Step S103: The electronic device determines a target model from a plurality of preset models based on the hearing level of the user.

Hearing levels are in a one-to-one correspondence with the preset models. As shown in FIG. 8, each hearing level corresponds to one preset model. For example, a preset model corresponding to the hearing level 2 is a preset model 2. When the electronic device determines that the hearing level of the user is the hearing level 2, the electronic device may determine the preset model 2 as the target model.

For example, assuming that the hearing test in step S101 includes five volumes, five preset models may be preset in the electronic device. The five preset models respectively correspond to hearing levels corresponding to the five volumes. Further, when the user selects one of the volumes, the target model is selected from the five preset models. A quantity of volumes in the hearing test of the electronic device and a quantity of preset models are not limited in embodiments of this application.

It may be understood that the different volumes are used to distinguish between hearing levels of users, and the hearing levels of the users are used to determine the preset models.

### Step S104: When detecting a user operation of answering a call, the electronic device turns on a top microphone and a bottom microphone of a mobile phone, to obtain a noise signal and a downlink signal.

Positions of the top microphone (that is, the top microphone) and the bottom microphone (the bottom microphone) may be shown in FIG. 4.

In some embodiments, when receiving the call, the electronic device may display a call interface. Further, when the user operation of answering the call is detected by the electronic device, the electronic device may play, in response to the user operation, the downlink signal by using the earpiece, and turn on the top microphone and the bottom microphone of the mobile phone. The electronic device may use a signal collected by the top microphone as the downlink signal, and use a signal collected by the bottom microphone as the noise signal.

It should be noted that, because the top microphone is located close to the earpiece, the signal collected by the top microphone is used as the collected downlink signal in this embodiment of this application.

### Step S105: The electronic device obtains a feature vector of at least one frame of downlink signal based on the noise signal and the downlink signal.

In some embodiments, the electronic device may calculate an energy difference between the downlink signal and a noise signal corresponding to the frame of downlink signal, and use the energy difference as the feature vector of the frame of downlink signal. The frame of downlink signal and the frame of noise signal are respectively signals collected by the top microphone and the bottom microphone at a same moment. The energy difference may be energy differences between the frame of downlink signal and the frame of noise signal in a plurality of frequency bands.

In an implementation, the electronic device may separately perform frame segmentation and windowing processing on the noise signal and the downlink signal that are collected by the microphones, to obtain M frames of noise signals and M frames of downlink signals, that is, M groups of audio signals. M is a positive integer, and each group of audio signals includes a downlink signal and a noise signal that are collected at a same moment. Further, the electronic device separately performs subband segmentation processing on the downlink signal and the noise signal in each group of audio signals, to obtain N signal subbands of the downlink signal and N signal subbands of the noise signal, where N is an integer greater than 1. Further, the electronic device calculates an energy difference between a noise signal and a downlink signal that correspond to each signal subband signal in each frame of audio signal, to obtain an N-dimensional feature vector corresponding to each group of audio signals. The subband segmentation processing is to divide one frame of signal into a plurality of signals in different frequency bands, and each subband signal is in a different frequency band.

It is assumed that the M groups of audio signals include an i^{th} group of audio signals. The following uses the i^{th} group of audio signals as an example to describe a process of calculating a feature vector.

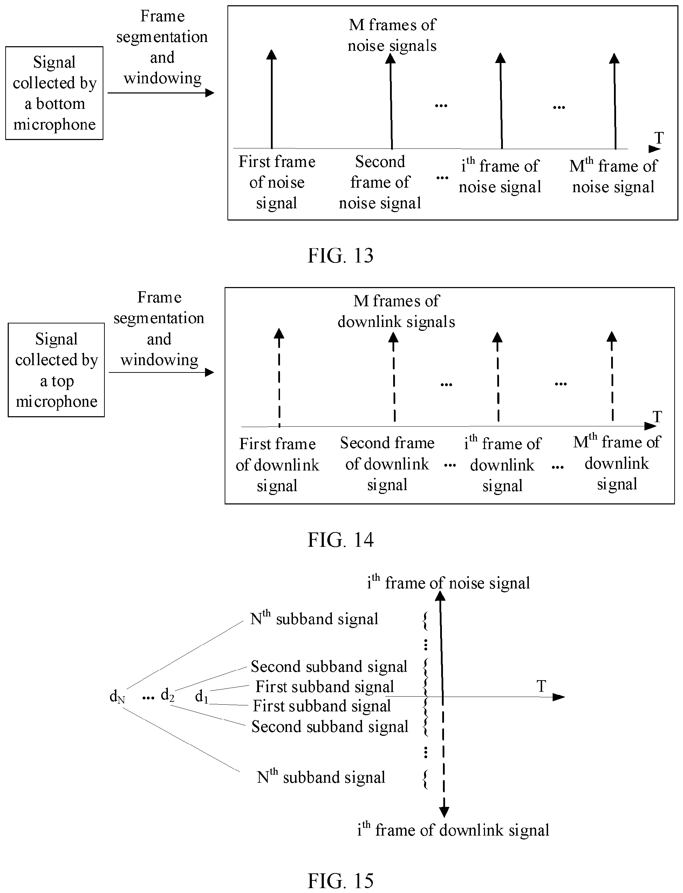

FIG. 13 is a diagram of performing frame segmentation and windowing on a signal collected by a bottom microphone according to an embodiment of this application. As shown in FIG. 13, after performing frame segmentation and windowing on the signal collected by the bottom microphone, the electronic device may obtain the M frames of noise signals. The M frames of noise signals include an i^{th} frame of noise signal, and i≤M.

FIG. 14 is a diagram of performing frame segmentation and windowing on a signal collected by a top microphone according to an embodiment of this application. As shown in FIG. 14, after performing frame segmentation and windowing on the signal collected by the top microphone, the electronic device may obtain the M frames of downlink signals. The M frames of downlink signals include an i^{th} frame of downlink signal.

Further, as shown in FIG. 15, the electronic device may separately perform subband segmentation processing on the i^{th} frame of noise signal and the i^{th} frame of downlink signal, to obtain N subband signals corresponding to the i^{th} frame of noise signal and N subband signals corresponding to the i^{th} frame of downlink signal. A quantity of subbands of the i^{th} frame of noise signal is equal to a quantity of subbands of the i^{th} frame of downlink signal. A frequency band of a subband signal of the i^{th} frame of noise signal is the same as a frequency band of a subband signal of the i^{th} frame of downlink signal. For example, a frequency band of a first subband signal of the i^{th} frame of noise signal is the same as a frequency band of a first subband signal of the i^{th} frame of downlink signal, a frequency band of a second subband signal of the i^{th} frame of noise signal is the same as a frequency band of a second subband signal of the i^{th} frame of downlink signal, and by analogy, a frequency band of an N^{th} subband signal of the i^{th} frame of noise signal is the same as a frequency band of an N^{th} subband signal of the i^{th} frame of downlink signal.

Finally, the electronic device may perform subtraction on energy of subband signals, in a same frequency band, of the i^{th} frame of noise signal and the i^{th} frame of downlink signal. For example, energy of the first subband signal of the i^{th} frame of downlink signal is subtracted from energy of the first subband signal of the i^{th} frame of noise signal to obtain d₁, energy of the second subband signal of the i^{th} frame of downlink signal is subtracted from energy of the second subband signal of the i^{th} frame of noise signal to obtain d₂, and by analogy, energy of the N^{th} subband signal of the i^{th} frame of downlink signal is subtracted from energy of the N^{th} subband signal of the i^{th} frame of noise signal to obtain d_{N}. In this way, it may be obtained that an N-dimensional feature vector of the i^{th} frame of downlink signal is [d₁, d₂, ..., and d_{N}]^{T}. The superscript T represents transpose of a vector or a matrix.

### Step S106: The electronic device inputs the feature vector of the at least one frame of downlink signal into the target model, to obtain an output sound level.

Sound levels of the electronic device include the output sound level, and the sound level is used to adjust a play volume of the earpiece. The sound level may be a sound pressure level of the downlink signal, for example, in a unit of decibel. A higher sound level indicates a higher play volume of the earpiece. For example, volumes that may be obtained through adjustments by using a volume button of the mobile phone include 10 levels. That is, the user may press a volume up button 10 times to adjust the mobile phone from a lowest volume to a highest volume. The 10 levels may sequentially be a sound level 1, and a sound level 2, to a sound level 10, and the output sound level is one of the sound level 1 to the sound level 10. The sound level 1 is a lowest level with a lowest play volume. The sound level 10 is a highest level with a highest play volume.

In some embodiments, when the feature vector of the at least one frame of downlink signal is a feature vector of one frame of downlink signal, the electronic device may input the feature vector of the frame of downlink signal and a sound level of the earpiece at previous several moments of the frame of downlink signal into the target model, to obtain the output sound level. For example, if the feature vector of the at least one frame of downlink signal is the feature vector of the i^{th} frame of downlink signal, namely, [d₁, d₂, ..., and d_{N}]^{T}, the electronic device may input the feature vector of the i^{th} frame of downlink signal into the target model, to obtain the output sound level.

In some other embodiments, if the feature vector of the at least one frame of downlink signal includes feature vectors of a plurality of frames of downlink signals, the electronic device may first select a feature vector of one frame of downlink signal from the feature vectors of the plurality of frames of downlink signals, input the feature vector of the frame of downlink signal and a sound level of the earpiece at previous several moments of the frame of downlink signal into the target model, to obtain the output sound level.

The output sound level is an electronic device-predicted lowest sound level that the user is able to clearly hear or an electronic device-predicted comfortable sound level for the user in a current environment.

It should be noted that, in some embodiments, the user cannot clearly hear the voice played at the volume 1, but clearly hears the voice played at the volume 2. The user selects the volume 2. Correspondingly, the electronic device determines the preset model 2 as the target model. In this case, an output sound level of the preset model 2 is the lowest sound level that the user is able to clearly hear. In the method, downlink signals and noise signals collected by the electronic device in different environments are different, and feature vectors input to the target model are different. Therefore, the target model outputs different volumes. The output sound level is the lowest sound level that the user is able to clearly hear in the current environment. According to the method, information security of the user in the call process can be ensured, to avoid call content being heard by an outsider.

In some other embodiments, the user can clearly hear the voice played at the volume 1, but the user considers that the volume 2 sounds more comfortable and better meets a volume requirement of the user. In this case, an output sound level of the preset model 2 is the comfortable sound level for the user. In the method, downlink signals and noise signals collected by the electronic device in different environments are different, and feature vectors input to the target model are different. Therefore, the target model outputs different volumes. The output sound level is the comfortable sound level that the user is able to clearly hear in the current environment, which can improve user experience of the user in the call process.

Step S107: The electronic device plays the downlink signal based on the output sound level.

In some embodiments, after obtaining the output sound level, the electronic device compares the output sound level with a current sound level at which the electronic device plays the downlink signal, and adjusts the current sound level to the output sound level when a difference between the output sound level and the current sound level is greater than a preset threshold.

For example, the sound levels of the electronic device include 10 levels, which are sequentially the sound level 1 to the sound level 10 in ascending order, and a decibel difference of each sound level is 5 decibels. Assuming that the current sound level is the sound level 1, the output sound level is a sound level 3, and the preset threshold is two sound levels, the electronic device adjusts the current sound level from the sound level 1 to the sound level 3. That is, the electronic device may increase a decibel value of the downlink signal by 10 decibels, and then play the downlink signal by using the earpiece.

Volumes of downlink signals in various frequency bands are different at a same sound level.

FIG. 16 shows an example of downlink signals in some frequency bands. FIG. 16 shows an example of downlink signals in three frequency bands. Three triangles on vertical coordinates identify sound pressure levels of the downlink signals in the three frequency bands at the sound level 1. The three circles on the vertical coordinates identify sound pressure levels of the downlink signals in the three frequency bands at the sound level 1. As shown in FIG. 16, FIG. 16 shows an example in which volumes of the downlink signals in the three frequency bands are different at a same sound level.

As shown in FIG. 16, after the electronic device adjusts the current sound level from the sound level 1 to the sound level 3, a level of the sound level 3 is higher than a level of the sound level 1. Assuming that a difference of each sound level is 5 decibels, when the current sound level is adjusted from the sound level 1 to the sound level 3, a play volume of a downlink signal in each frequency band increases by 10 decibels. It may be understood that if the electronic device is a mobile phone, and volumes that may be obtained through adjustments by using a volume button of the mobile phone include 10 levels, automatically adjusting the current sound level from the sound level 1 to the sound level 3 by the mobile phone has same effect as manually pressing a volume up button twice by the user.

It should be noted that there may alternatively be 10 or 15 sound levels of the electronic device. This is not limited herein.

In some other embodiments, the electronic device may adjust the current sound level to the output sound level only when differences between the current sound level and a plurality of output sound levels in preset duration are all greater than the preset threshold. It may be understood that according to the method, negative impact caused by frequent volume adjustments due to data fluctuation can be avoided.

It should be noted that, the electronic device may play a same downlink signal at a same sound level at different play volumes for users at different hearing levels. For example, when a user at a hearing level 1 uses the electronic device, the electronic device plays a downlink signal in a frequency band A at the sound level 1, and in this case, a play volume of the downlink signal is the volume 1. When a user at a hearing level 2 uses the electronic device, the electronic device plays the downlink signal in the frequency band A at the sound level 1, and in this case, the play volume of the downlink signal is the volume 2. A decibel value of the volume 1 is not equal to a decibel value of the volume 2.

The following describes several application scenarios by using examples.
(1) A same user uses a same electronic device to make a call in different environments.

It is assumed that a user A selects, on the electronic device through the hearing level test described above, a lowest volume at which the user A is able to clearly hear as a satisfactory volume, and a preset model corresponding to the satisfactory volume is a target model. In this case, the target model is configured to output, in different environments, the lowest sound level that the user A is able to clearly hear, and the electronic device may adjust, in the different environments, a play volume of an earpiece to the lowest volume at which the user is able to clearly hear.

When the user A makes a call in the different environments, because magnitudes of noise signals in the environments are different, feature vectors obtained by the electronic device in different scenarios are different. The electronic device inputs the different feature vectors into the target model, to obtain different output sound levels.

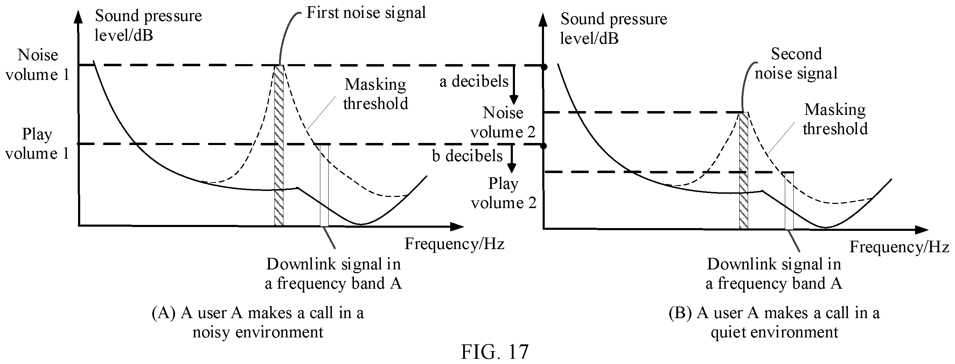

FIG. 17 is a diagram in which an output sound level changes with an environment according to an embodiment of this application.

As shown in (A) in FIG. 17, it is assumed that when the user A makes a call through the electronic device in a noisy environment, for example, in an environment such as a canteen or a road, a noise signal with a highest decibel value is a first noise signal, and a volume of the first noise signal is a noise volume 1. In this case, the electronic device may obtain a sound level 1 based on the target model. The sound level 1 is specifically used to adjust play volumes (decibel values) of downlink signals in various frequency bands. In FIG. 17, a downlink signal in a frequency band A is used as an example for description. After the electronic device adjusts a sound level of the earpiece to the sound level 1, a play volume of the downlink signal in the frequency band A is a play volume 1. It can be learned that a decibel value of the play volume 1 is greater than a masking threshold caused by the first noise signal at this time.

As shown in (B) in FIG. 17, it is assumed that when the user A makes a call through the electronic device in a quiet environment, for example, in an environment such as a conference room, a noise signal with a highest decibel value is a second noise signal, and a volume of the second noise signal is a noise volume 2. In this case, the electronic device may obtain a sound level 2 based on the target model, and the sound level 2 is specifically used to adjust play volumes of downlink signals in various frequency bands. In FIG. 17, a downlink signal in a frequency band A is used as an example for description. After the electronic device adjusts a sound level of the earpiece to the sound level 1, a play volume of the downlink signal in the frequency band A is a play volume 2. It can be learned that a decibel value of the play volume 2 is greater than a masking threshold caused by the second noise signal at this time.

From comparison between (A) and (B) in FIG. 17, it can be learned that, the noise volume 2 of the second noise signal is a decibels lower than the noise volume 1 of the first noise signal, and the play volume 2 is b decibels lower than the play volume 1. Both the play volume 1 and the play volume 2 are higher than the masking thresholds in the environments at that time. It can be learned that the play volume obtained based on the target model when the user A is in the quiet environment is lower than the play volume obtained based on the target model when the user A is in the noisy environment. In other words, the electronic device may lower the play volume of the downlink signal based on the target model in the quiet environment, increase the play volume of the downlink signal in the noisy environment, and keep the play volume of the downlink signal constantly higher than the masking threshold, so that the play volume of the downlink signal is a lowest volume at which the user is able to clearly hear in any environment.

(2) Different users use different electronic devices to make calls in a same environment.

It is assumed that a user A is in an age group 1, the user A selects, through the hearing level test described above, a lowest volume (for example, a volume 3) that the user A is able to clearly hear as a satisfactory volume, and a preset model corresponding to the satisfactory volume is a preset model 3; and the user B is in an age group 2, the user B selects, through the hearing level test described above, a lowest sound level (for example, a volume 1) that the user B is able to clearly hear as a satisfactory volume, and a preset model corresponding to the satisfactory volume is a preset model 1. The volume 3 is greater than the volume 1.

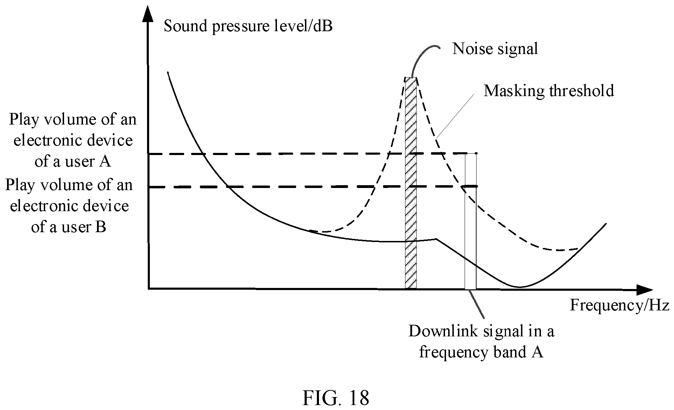

FIG. 18 shows an example of a downlink signal in a frequency band A. As shown in FIG. 18, when the user A and the user B make calls in the same environment, a decibel value of a play volume obtained by an electronic device of the user A based on the preset model 3 is greater than a decibel value of a play volume obtained by the user B based on the preset model 1. Both the play volume of the electronic device of the user A and the play volume of an electronic device of the user B are greater than a masking threshold.

It can be learned that the user may select, through the hearing level test, a preset model that meets a hearing requirement of the user. Correspondingly, the electronic device may obtain, based on the preset model selected by the user, a sound level that meets the hearing requirement of the user, and adjust a play volume of the downlink signal to a play volume corresponding to the sound level, to meet hearing requirements of different groups.

(3) A user uses a same electronic device to make a call in two environments with a large noise difference.

In some embodiments, the electronic device may slowly adjust a volume by using a target model when the user is separately in the two environments with the large noise difference. For the target model, refer to the foregoing description. Details are not described herein again.

It should be noted that the following uses a call process as an example for description, which is also applicable to another scenario such as a scenario of music playing.

For example, in the call process, the user moves from a quiet environment to a noisy environment, and the electronic device may slowly increase a sound level at which an earpiece plays a downlink signal, until the sound level of the earpiece is increased to a sound level corresponding to the noisy environment. For another example, in the call process, the user moves from the noisy environment to the quiet environment, and the electronic device may slowly lower the sound level at which the earpiece plays the downlink signal, until the sound level of the earpiece is lowered to a sound level corresponding to the quiet environment.

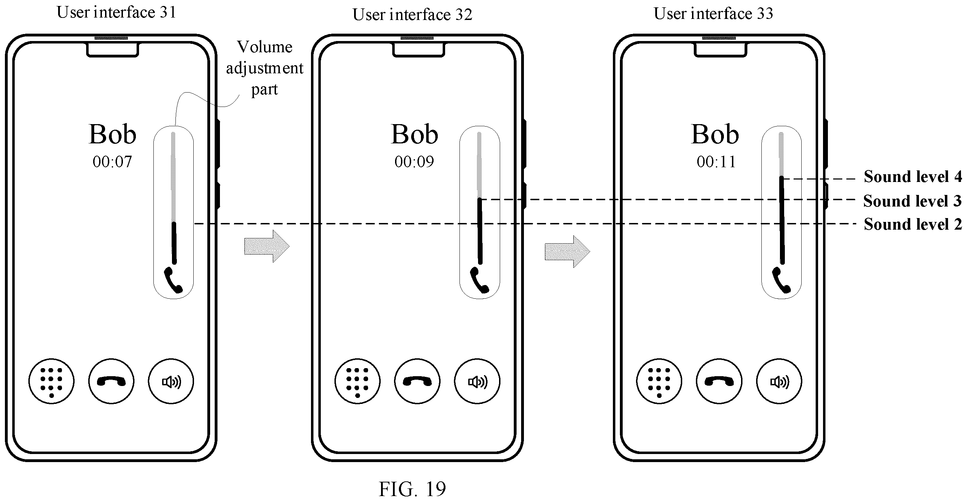

FIG. 19 shows an example of a volume adjustment process in a call process of a user. A user interface 31, a user interface 32, and a user interface 33 shown in FIG. 19 are interface diagrams displayed on the electronic device of the user during a call between the user and Bob (Bob). The user interface 31 is a user interface at 00:07 of the call, the user interface 32 is a user interface at 00:09 of the call, and the user interface 33 is a user interface at 00:11 of the call.

Because the user is in the quiet environment at 00:07 of the call, the electronic device may obtain a sound level 2 by using a target model based on a downlink signal and a noise signal that are collected in the environment. It can be learned from the user interface 31 that the electronic device plays the downlink signal at the sound level 2 at 00:07 of the call made by the user.

After 00:07 of the call made by the user, the environment becomes noisy. For example, when the user moves a position or music starts to be played in a current environment, the electronic device obtains a sound level 4 by using the target model based on a downlink signal and a noise signal that are collected in the noisy environment. The electronic device may first adjust the play sound level of the earpiece to a sound level 3 at 00:09 of the call, as shown in the user interface 32; and then adjust the play sound level of the earpiece to the sound level 4 at 00: 11 of the call, as shown in the user interface 33. It may be understood that according to the method, a case in which the user feels uncomfortable in hearing when volumes at a plurality of levels are obtained through sudden adjustments can be avoided.

It should be noted that the user interface 31 to the user interface 33 are merely example interfaces provided in this embodiment of this application, and an objective of the user interface 31 to the user interface 33 is merely to clearly display a sound level of a current interface, and should not constitute a limitation on embodiments of this application. In an actual volume adjustment process of the electronic device, volume adjustment parts in the user interface 31 to the user interface 33 in FIG. 19 may not be displayed. That is, the electronic device may not display the sound level of the earpiece during an automatic volume adjustment.

(4) A user operates different applications on a same electronic device.

In some embodiments, the different applications on the electronic device may correspond to different target models. Further, the electronic device may perform, based on a target model corresponding to an application, a volume adjustment on audio played by the application. For example, a call application and a music application are installed on the electronic device. The call application corresponds to a first target model, and the music application corresponds to a second target model. In this case, when the user makes a call in a same environment by using the call application, a play volume of a voice of the other party in the call during the call is obtained based on the first target model. When the music application is used for play, a volume of a song played by the music application is obtained based on the second target model. The play volume of the voice of the other party in the call and the volume of the song may be different volumes.

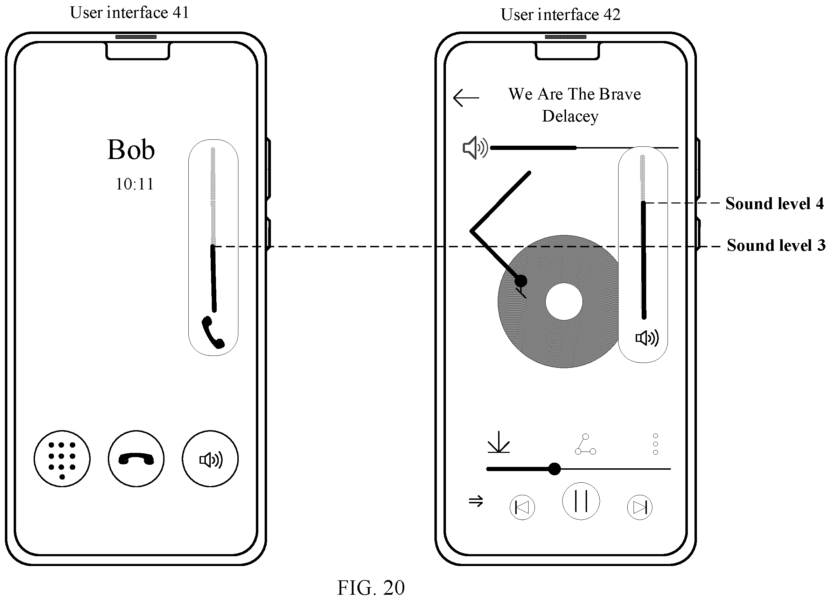

FIG. 20 shows an example of sound levels obtained through adjustments performed by an electronic device when a user uses different applications through the same electronic device. The following uses the call application and the music application as examples. It is assumed that a first target application corresponding to the call application is a preset model corresponding to a lowest volume at which the user is able to clearly hear, and a second target application corresponding to the music application is a preset model corresponding to a most comfortable volume for the user. It may be understood that the user may consider that confidentiality of call content is higher than that of music, and therefore expects that a volume during a call is low to a greatest extent.

As shown in FIG. 20, a sound level, of a play volume of an earpiece, obtained by the electronic device based on a collected downlink signal and a collected noise signal by using the first target application is a sound level 3. That is, as shown in a user interface 41, the electronic device plays a voice of the other party Bob in the call at the sound level 3. In a same environment condition as that of the user interface 41, a sound level, of a play volume of a song, obtained by the electronic device based on a collected downlink signal and a collected noise signal by using the second target application is a sound level 4. That is, as shown in a user interface 42, the electronic device plays the song in the music application at the sound level 4. In other words, when the user plays audio by using the different applications in the same environment through the electronic device, volumes of the audio heard by the user are different. For example, a volume of a voice, of the other party, that is played by the earpiece and that is heard during a call in the same environment is lower than a volume of music in a headset heard during use of the music application in the same environment.

It should be noted that the user interface 41 and the user interface 41 are merely example interfaces provided in this embodiment of this application, and an objective of the user interface 41 and the user interface 41 is merely to clearly display a sound level of a current interface, and should not constitute a limitation on embodiments of this application. In other words, in an actual volume adjustment process of the electronic device, volume adjustment parts in the user interface 41 and the user interface 42 in FIG. 20 may not be displayed. That is, the electronic device may not display the sound level during an automatic volume adjustment.

(5) An electronic device may adjust a target model based on a user habit.

In some embodiments, the electronic device may record, in an actual use process of a user, user data each time the user manually adjusts a volume. For example, the data may include a feature vector, a sound level obtained through the adjustment, a sound level at previous several moments of the adjustment, and the like. Further, the electronic device uses the user data as training data to train the target model, so that the target model conforms to the user habit.

The following uses a call process as an example. It is assumed that the electronic device automatically adjusts the volume in the call process of the user by using the target model, but the user manually lowers a sound level for a plurality of times.

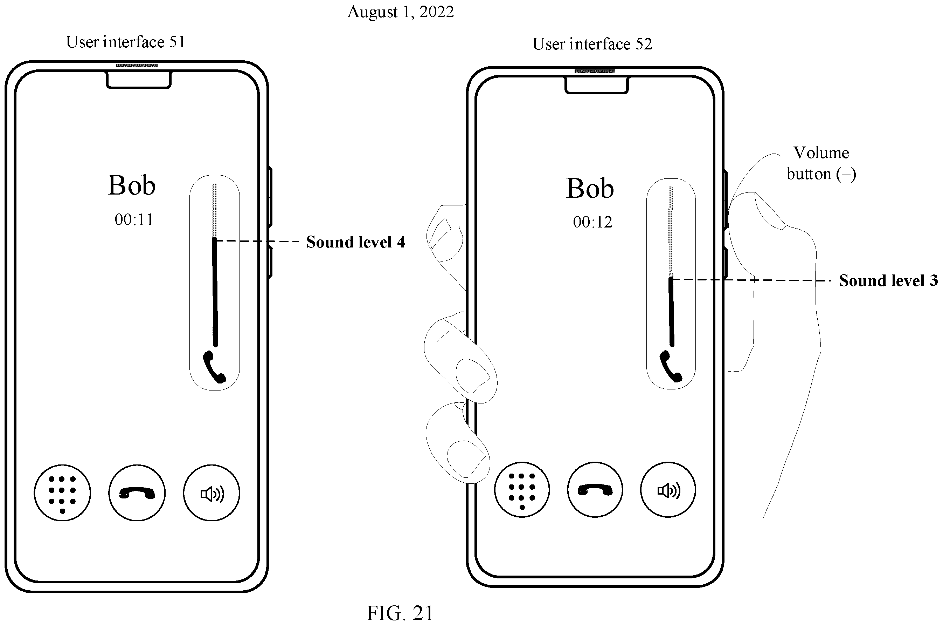

FIG. 21 shows an example of some application interfaces on which the user makes a call with Bob through the electronic device in a first environment condition on August 1, 2022. As shown in FIG. 21, a user interface 51 shows an example of a sound level 4 obtained through an adjustment performed by the electronic device by using the target model in the first environment condition. Because the user considers that a volume is large, as shown in a user interface 52, the user manually presses a volume down (-) button in a volume button, and the electronic device adjusts, to a sound level 3 in response to the user operation, a volume of a downlink signal played by an earpiece.

The electronic device may determine a piece of training data based on user data corresponding to the scenario shown in FIG. 21, for example, a feature vector determined at that time, a sound level obtained through the adjustment, and a sound level at previous several moments of the adjustment, and train the target model based on the training data.

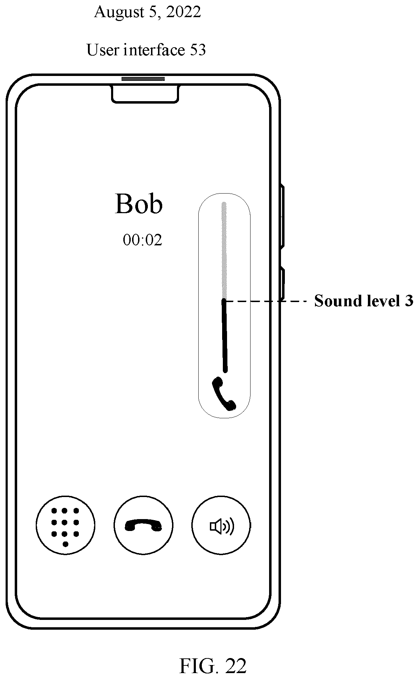

It is assumed that the electronic device trains the target model based on at least one piece of training data between August 1, 2022 and August 5, 2022, to obtain a trained target model. On August 5, 2022, the user makes a call with Bob again in the first environment condition through the electronic device, and the electronic device may obtain the sound level 3 based on the trained target model. As shown in FIG. 22, at 00:02 of the call between the user and Bob, the electronic device has adjusted a play volume of the earpiece to the sound level 3. Therefore, the user may not need to manually adjust the volume. It may be understood that the user may find, based on use experience, that a quantity of times the user manually adjusts the volume gradually decreases, and a play volume of the electronic device increasingly conforms to a use habit of the user.

It should be noted that the foregoing five application scenarios may be combined as required or separately appear in a use process of an electronic device that uses the volume adjustment method in embodiments of this application. This is not limited herein.

An embodiment of this application further provides an electronic device. The electronic device includes one or more processors and one or more memories. The one or more memories are coupled to the one or more processors. The one or more memories are configured to store computer program code. The computer program code includes computer instructions. When the one or more processors execute the computer instructions, the electronic device is enabled to perform the method described in the foregoing embodiments.

An embodiment of this application provides a computer program product including instructions. When the computer program product is run on an electronic device, the electronic device is enabled to perform the method described in the foregoing embodiments.

An embodiment of this application further provides a computer-readable storage medium, including instructions. When the instructions are run on an electronic device, the electronic device is enabled to perform the method described in the foregoing embodiments.

It can be understood that implementations of this application may be randomly combined, to achieve different technical effects.

All or some of the foregoing embodiments may be implemented by using software, hardware, firmware, or any combination thereof. When software is used to implement the embodiments, all or a part of the embodiments may be implemented in a form of computer program product. The computer program product includes one or more computer instructions. When the computer program instructions are loaded and executed on a computer, the procedure or functions according to this application are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state disk (Solid-State Disk)), or the like.

Persons of ordinary skill in the art may understand that all or some of the procedures of the methods in the foregoing embodiments may be implemented by a computer program by instructing related hardware. The program may be stored in the computer-readable storage medium. When the program is executed, the procedures in the foregoing method embodiments may be performed. The foregoing storage medium includes any medium that can store program code, such as a ROM, a random access memory RAM, a magnetic disk, or an optical disc.

In summary, the foregoing descriptions are merely embodiments of the technical solutions of this application, but are not intended to limit the protection scope of this application. Any modification, equivalent replacement, improvement, or the like made based on the disclosure of this application shall fall within the protection scope of this application.

## Claims

1. A volume adjustment method, applied to an electronic device, wherein the electronic device comprises a sound-emitting apparatus and at least one microphone, and the method comprises:
in response to a user operation of starting a hearing test, separately playing, by the electronic device, preset audio at a plurality of volumes, wherein the plurality of volumes comprise a first volume;
determining, by the electronic device based on a confirmation operation for the first volume, that a hearing level of a user is a first hearing level corresponding to the first volume;
when playing a downlink signal by using the sound-emitting apparatus, collecting, by the electronic device, the downlink signal and an ambient noise signal by using the at least one microphone; and
adjusting, by the electronic device, a play volume of the sound-emitting apparatus from a first sound level to a second sound level based on the first hearing level when at least one of the collected downlink signal and the collected ambient noise signal changes.

2. The method according to claim 1, wherein a higher first volume indicates a higher level of the second sound level.

3. The method according to claim 1 or 2, wherein the first volume is a comfortable volume for the user or a lowest volume at which the user is able to clearly hear in a current environment.

4. The method according to any one of claims 1 to 3, wherein greater energy of a changed ambient noise signal indicates a higher level of the second sound level.

5. The method according to any one of claims 1 to 4, wherein the adjusting, by the electronic device, a play volume of the sound-emitting apparatus from a first sound level to a second sound level when at least one of the downlink signal and the ambient noise signal changes comprises:
adjusting, by the electronic device, the play volume of the sound-emitting apparatus to the second sound level when a difference between the first sound level and the second sound level equals a first preset quantity of levels.

6. The method according to any one of claims 1 to 5, wherein the adjusting, by the electronic device, a play volume of the sound-emitting apparatus from a first sound level to a second sound level when at least one of the downlink signal and the ambient noise signal changes comprises:
adjusting, by the electronic device, the play volume of the sound-emitting apparatus to a third sound level when the difference between the first sound level and the second sound level equals a second preset quantity of levels, wherein the third sound level is higher than the first sound level and lower than the second sound level.

7. The method according to any one of claims 1 to 6, wherein the method further comprises:
in response to a user operation for a first application, playing, by the electronic device in a first environment condition at a second volume by using the sound-emitting apparatus, a downlink signal corresponding to the first application; and
in response to a user operation for a second application, playing, by the electronic device in the first environment condition at a third volume by using the sound-emitting apparatus, a downlink signal corresponding to the second application, wherein
the second volume is not equal to the third volume.

8. The method according to claim 7, wherein the first application is a call-type application, the second application is a music-type application, and the second volume is greater than the third volume.

9. The method according to any one of claims 1 to 8, wherein the method further comprises:
determining, by the electronic device, a preset model corresponding to the first hearing level as a target model, wherein the target model is obtained through training based on user data of a user that meets the first hearing level, and the user data comprises a target sound level of the user at the first hearing level in a second environment condition and an energy difference between a downlink signal and an ambient noise signal that are collected in the second environment condition; and
inputting, by the electronic device, an energy difference between a changed downlink signal and the changed ambient noise signal into the target model, to obtain the second sound level.

10. The method according to claim 9, wherein the changed downlink signal comprises N first subband signals, the changed ambient noise signal comprises the N second subband signals, N is a positive integer, the N first subband signals are in a one-to-one correspondence with the N second subband signals, and a first subband signal and a second subband signal that correspond to each other form a group of signals; and
the energy difference between the changed downlink signal and the changed ambient noise signal comprises energy differences of N groups of signals.

11. The method according to claim 9 or 10, wherein the user data further comprises a play volume of the sound-emitting apparatus at previous several moments of the target sound level, and the inputting, by the electronic device, an energy difference between a changed downlink signal and the changed ambient noise signal into the target model, to obtain the second sound level comprises:
inputting, by the electronic device, the energy difference between the changed downlink signal and the changed ambient noise signal and the play volume of the sound-emitting apparatus at the previous several moments into the target model, to obtain the second sound level.

12. The method according to claims 9 to 11, wherein after the adjusting the play volume of the sound-emitting apparatus to the second sound level, the method further comprises:
in response to an operation of adjusting a volume button by the user, adjusting, by the electronic device, the play volume of the sound-emitting apparatus to the target sound level;
storing, by the electronic device, the target sound level and the energy difference between the changed downlink signal and the changed ambient noise signal; and
training, by the electronic device, the target model by using the target sound level and the energy difference between the changed downlink signal and the changed ambient noise signal as sample data.

13. The method according to any one of claims 1 to 12, wherein the at least one microphone comprises a first microphone and a second microphone, a distance between the first microphone and the sound-emitting apparatus is less than a distance between the second microphone and the sound-emitting apparatus, the first microphone is configured to collect the downlink signal, and the second microphone is configured to collect the ambient noise signal.

14. A volume adjustment apparatus, wherein the apparatus comprises a processing module, a sound-emitting apparatus, and at least one microphone, wherein
the sound-emitting apparatus is configured to separately play preset audio at a plurality of volumes, wherein the plurality of volumes comprise a first volume;
the processing module is configured to determine, based on a confirmation operation for the first volume, that a hearing level of a user is a first hearing level corresponding to the first volume;
the at least one microphone is configured to: when the sound-emitting apparatus plays a downlink signal, collect the downlink signal and an ambient noise signal; and
the sound-emitting apparatus is configured to adjust a play volume from a first sound level to a second sound level based on the first hearing level when at least one of the collected downlink signal and the collected ambient noise signal changes.

15. The apparatus according to claim 14, wherein a higher first volume indicates a higher level of the second sound level.

16. The apparatus according to claim 15, wherein the first volume is a lowest volume at which the user is able to clearly hear or a comfortable volume for the user in a current environment.

17. The apparatus according to any one of claims 14 to 16, wherein greater energy of a changed ambient noise signal indicates a higher level of the second sound level.

18. The apparatus according to any one of claims 14 to 17, wherein the sound-emitting apparatus is specifically configured to play the downlink signal at the second sound level when a difference between the first sound level and the second sound level equals a first preset quantity of levels.

19. The apparatus according to any one of claims 14 to 18, wherein the sound-emitting apparatus is specifically configured to: play the downlink signal at the third sound level when the difference between the first sound level and the second sound level equals a second preset quantity of levels, wherein the third sound level is higher than the first sound level and lower than the second sound level.

20. The apparatus according to any one of claims 14 to 19, wherein the sound-emitting apparatus is configured to play, in a first environment condition at a second volume, a downlink signal corresponding to the first application; and
the sound-emitting apparatus is configured to play, in the first environment condition at a third volume, a downlink signal corresponding to the second application, wherein the second volume is not equal to the third volume.

21. An electronic device, wherein the electronic device comprises one or more processors and one or more memories, the one or more memories are coupled to the one or more processors, the one or more memories are configured to store computer program code, the computer program code comprises computer instructions, and when the one or more processors execute the computer instructions, the electronic device is enabled to perform the method according to any one of claims 1 to 13.

22. A computer-readable storage medium, comprising instructions, wherein when the instructions are run on an electronic device, the electronic device is enabled to perform the method according to any one of claims 1 to 13.
